# EUROPEAN PATENT APPLICATION

(11) **EP 2 359 852 A1**
(43) Date of publication of application: **24.08.2011**
(21) Application number: 09826195.1
(22) Date of filing: 17.11.2009
(51) Int. Cl.: A61K 39/395, A61K 9/127, A61K 31/4745, A61K 45/00, A61K 45/06, A61K 47/42, A61K 47/48, A61P 35/00

(54) **NOVEL CANCER TARGETING THERAPY USING COMPLEX OF SUBSTANCE CAPABLE OF BINDING SPECIFICALLY TO CONSTITUENT FACTOR OF CANCER STROMA AND ANTI-TUMOR COMPOUND**

(30) Priority: 17.11.2008 JP 2008293930; 25.05.2009 JP 2009125871
(71) Applicant: Japan Health Sciences Foundation, Tokyo 103-0001 (JP); Riken, Wako-shi, Saitama 351-0198 (JP)
(72) Inventor: MATSUMURA, Yasuhiro, Kashiwa-shi Chiba 277-8577 (JP); YASUNAGA, Masahiro, Kashiwa-shi Chiba 277-8577 (JP); MANABE, Shino, Wako-shi Saitama 351-0198 (JP)
(74) Representative: Woods, Geoffrey Corlett
(86) International application number: PCT/JP2009/069509
(87) International publication number: WO 2010/055950

(57) **Abstract**

The present invention is intended to provide a pharmaceutical for tumor treatment that stays specifically in interstitium for a long time and exhibits an effect, and provides a complex consisting of a substance having specific binding affinity for stroma and an antitumor compound bound to the substance via a linker.

## Description

### Technical Field

The present invention relates to a use of a substance having specific binding affinity for stroma for delivering an antitumor compound to a tumor site. Specifically, the present invention relates to a complex of a substance having specific binding affinity for stroma and an antitumor site (e.g., an antitumor compound, a functional structure capable of sustained release of an antitumor compound) and a use application thereof.

### Background Art

From the last half of the 1970s, when a method of generating monoclonal antibodies was established, research into "missile therapy", which is to selectively attack tumor lesions using a monoclonal antibody with a toxin, an antitumor compound or the like added thereto, became active (patent documents 1-4); later, however, except for approval for some forms of lymphoma and leukemia (non-patent documents 1 and 2), clinical utility of missile therapy for ordinary solid cancers such as lung cancer, colorectal cancer, breast cancer, and gastric cancer has not been demonstrated to date (non-patent documents 3-11). Problems with what is called missile therapy using a monoclonal antibody against a tumor-specific antigen include:
(1) the limited applicability of monoclonal antibodies to tumors with a specific antigen expressed on the membrane surface of the tumor cells,
(2) the possibility that the monoclonal antibody gets neutralized by an antigen released in the blood and cannot be delivered to the tumor focus of interest,
(3) the possibility that even if the monoclonal antibody is successfully delivered to the tumor focus, the interstitium hampers the monoclonal antibody to reach where tumor cells of interest are present, until reaching tumor cells from tumor blood vessels after leakage from tumor blood vessels, and the like.

Of tumors, in particular, intractable cancers such as pancreatic cancer, scirrhous gastric cancer, colorectal cancer, and lung cancer are known to be rich in interstitium. Although collagens, which are abundant in interstitium, are also present in normal living organisms, they are well developed in inflamed tissues and tumor tissues having tissue systems similar thereto (non-patent document 12). Type IV collagen is abundant around tumor blood vessels, whereas type I and type III collagens are prevalent between tumor cells and tumor blood vessels. The degree thereof widely differs depending on the kind of tumor; generally, intractable cancers for which drugs are unlikely to be effective are particularly rich in interstitium represented by collagen. In particular, it is known that human tumor tissues are richer in interstitium than mouse tumor tissues.

Matsumura found an EPR effect (enhanced permeation and retention effect) wherein tumors are angiologically characterized by accentuated tumor vascular permeability and allow macromolecular substances unlikely to leak from normal blood vessels to readily leak from tumor blood vessels, and macromolecular substances that have once leaked from blood vessels in tumor tissue cannot be drained to lymph vessels but stay long in the tumor tissue because of a lack of lymphangiogenesis over vasculogenesis (non-patent document 13).

Although the antitumor compound SN-38 is already in clinical applications as a prodrug called CPT-11, it cannot be distributed with distinguishing between normal tissue and tumor tissue because of its identity as a small molecule, nor is it possible to allow it to long stay in tumor. Therefore, the feature of the active entity SN-38 of having a time-dependent antitumor effect is not brought into the best use, and the problem of intense manifestation of adverse reactions has been pointed out clinically (non-patent documents 14-17).

### [Prior Art Documents]

### [patent documents]

patent document 1: JP-A-2007-39346
patent document 2: National Publication of International
   Patent Application No. 2008-501029
patent document 3: National Publication of International
   Patent Application No. 2007-512324
patent document 4: National Publication of International
   Patent Application No. 2008-524202

### [non-patent documents]

non-patent document 1: Bross PF et al. Approval summary: gemtuzumab ozogamicin in relapsed acute myeloid leukemia. Clin Cancer Res 2001: 7:1490-1496
non-patent document 2: Allen TM. Ligand-targeted therapeutics in anticancer therapy. Nature Rev Cancer 2006: 2: 750-763
non-patent document 3: Omelyanenko V et al. HPMA copolymer-anticancer drug-OV-TL16 antibody conjugates. 1. influence of the method of synthesis on the binding affinity to OVCAR-3 ovarian carcinoma cells in vitro. J Drug Targeting 1996; 3: 357-73
non-patent document 4: Gilliland DG et al. Antibody-directed cytotoxic agents: use of monoclonal antibody to direct the action of toxin A chains to colorectal carcinoma cells. Proc Natl Acad Sci U S A. 1980 Aug;77(8):4539-43
non-patent document 5: Blythman HE et al. Immunotoxins: hybrid molecules of monoclonal antibodies and a toxin subunit specifically kill tumour cells. Nature. 1981 Mar 12;290(5802):145-6
non-patent document 6: Tsukada Y et al. Chemotherapy by intravenous administration of conjugates of daunomycin with monoclonal and conventional anti-rat alpha-fetoprotein antibodies. Proc Natl Acad Sci U S A. 1982 Dec;79(24):7896-9
non-patent document 7: Hurwitz E et al. A conjugate of adriamycin and monoclonal antibodies to Thy-1 antigen inhibits human neuroblastoma cells in vitro. Ann N Y Acad Sci. 1983;417:125-36 non-patent document 8: Gallego J et al. Preparation of four daunomycin-monoclonal antibody 791T/36 conjugates with antitumour activity. Int J Cancer. 1984 Jun 15;33(6):737-44
non-patent document 9: Spearman ME et al. Disposition of the monoclonal antibody-vinca alkaloid conjugate KS1/4-DAVLB (LY256787) and free 4-desacetylvinblastine in tumor-bearing nude mice. J Pharmacol Exp Ther. 1987 May;241(2):695-703
non-patent document 10: Braslawsky GR et al. Adriamycin(hydrazone)-antibody conjugates require internalization and intracellular acid hydrolysis for antitumor activity. Cancer Immunol Immunother. 1991;33(6):367-74
non-patent document 11: Doronina SO et al. Development of potent monoclonal antibody auristatin conjugates for cancer therapy. Nat Biotechnol. 2003 Jul;21(7):778-84. Epub 2003 Jun 1
non-patent document 12: Dvorak HF. Tumors: Wounds that do not heal. Similarities between tumor stroma generation and wound healing. New Engl J Med 1986 Dec 25; 315(26): 1650-9
non-patent document 13: Matsumura Y, Maeda H. A new concept for macromolecular therapeutics in cancer chemotherapy: mechanism of tumoritropic accumulation of proteins and the antitumor agent SMANCS. Cancer Res. 1986 Dec;46(12 Pt 1):6387-92
non-patent document 14: Koizumi F et al. Novel SN-38-incorporating polymeric micelles, NK012, eradicate vascular endothelial growth factor-secreting bulky tumos. Cancer Res 2006 Oct 15; 66(20): 10048-56
non-patent document 15: Sumitomo M et al. Novel SN-38-incorporated polymeric micelles, NK012, strongly suppress renal cancer progression. Cancer Res. 2008: 122: 2148-2153
non-patent document 16: Saito Y et al. Enhanced distribution of NK012 and prolonged sustained-release of SN-38 within tumors are key strategic point for a hypovascular tumor. Cancer Sci. 2008: 90: 1258-1264
non-patent document 17: Matsumura Y. Poly (amino acid) micellar nanocarriers in preclinical and clinical studies. Adv. Drug Del. Rev. 2008: 60: 899-914

### [SUMMARY OF THE INVENTION]

### Problems to be Solved by the Invention

The present invention is intended to provide a complex that stays specifically in interstitium for a long time and acts on tumor blood vessels and/or tumor cells to have an antitumor effect.

### Means of Solving the Problems

While recognizing the suggested possibility that interstitium, represented by collagen, is abundantly present around tumors and may inhibit the access of antitumor compounds to tumor cells, the present inventors extensively investigated to solve the above-described problems and found that when using a complex of a substance having specific binding affinity for stroma and an antitumor site (e.g., antitumor compounds, functional structures capable of sustained release of an antitumor compound), better antitumor action is exhibited by acting on tumor blood vessels and/or tumor cells, than a complex of an antibody against an antigen on the tumor cell surface and an antitumor compound, and have developed the present invention.

Accordingly, the present invention provides the following:
[1] A complex comprising a substance having specific binding affinity for stroma and an antitumor site bound to the substance.
[2] The complex described in [1], wherein the substance having specific binding affinity for stroma and the antitumor site are bound via a linker.
[3] The complex described in [1], wherein the antitumor site is an antitumor compound.
[4] The complex described in [1], wherein the antitumor site is a functional structure capable of sustained release of an antitumor compound.
[5] The complex described in [4], wherein the functional structure capable of sustained release of an antitumor compound is a liposome or micelle containing an antitumor compound.
[6] The complex-described in [1], wherein the substance having specific binding affinity for stroma is an antibody having specific binding affinity for stroma or a binding fragment thereof.
[7] The complex described in [1], wherein the stroma is a collagen.
[8] The complex described in [7], wherein the collagen is type IV collagen.
[9] The complex described in [1], wherein the stroma is fibrin.
[10] The complex described in [2], wherein the linker and the antitumor site are bound via an ester bond or a carbamate bond.
[11] The complex described in [2], wherein the linker and the antitumor site are bound via a carbonate bond or a thiocarbamate bond.
[12] A tumor therapeutic agent comprising the complex described in [1].
[13] An inhibitor of tumor blood vessel formation comprising the complex described in [1].
[14] A method of treating a tumor in a mammal, comprising administering an effective amount of the complex described in [1] to the mammal.
[15] A method of inhibiting the formation of tumor blood vessels in a mammal, comprising administering an effective amount of the complex described in [1] to the mammal.
[16] The complex described in [1], wherein the complex is to be used in the treatment of a tumor.
[17] The complex described in [1], wherein the complex is to be used in the inhibition of the formation of tumor blood vessels.

### Effect of the Invention

According to the present invention, a complex is provided that stays specifically in interstitium for a long time to exhibit an excellent antitumor effect. In particular, using the complex of the present invention makes it possible to allow an antitumor compound having a time-dependent antitumor effect to effectively exhibit the antitumor effect. Also, because the complex of the present invention acts on tumor blood vessels and/or tumor cells, a remarkably higher antitumor effect is expectable than conventional complexes.

### Brief Description of the Drawings

Fig. 1 is a drawing showing a typical human pancreatic cancer tissue.
Fig. 2 shows results of an analysis of the expression of EpCAM on tumor cell surfaces by flow cytometry.
Fig. 3 is a drawing showing an in vivo imaging of antibodies.
Fig. 4 is a schematic diagram showing the binding of an SN-38-polymer compound and an antibody.
Fig. 5 is a drawing showing the in vitro cytocidal effects of various complexes.
Fig. 6 is a drawing showing the in vivo antitumor effects of various complexes.
Fig. 7 is a drawing showing the histopathological profiles of tumors receiving and not receiving an administration of an antimouse type IV collagen antibody-SN-38 complex.
Fig. 8 is a drawing showing alignment of the cDNA sequence of the VH portion of the hybridoma clone 35-4 and the corresponding cDNA sequence of the rIgG2a clone BC088240.1.
Fig. 9 is a drawing showing alignment of a putative amino acid sequence of the VH portion of hybridoma clone 35-4 and the corresponding amino acid sequence of the rIgG2a clone BC088240.1.
Fig. 10 is a drawing showing alignment of the cDNA sequences of the VH portions of the hybridoma clones 6-1, 6-2 and 56P-1 and the corresponding cDNA sequences of the IgM variable region clone J00529.1 and the IgM constant region clone V00827.1.
   Fig. 11 is a drawing showing alignment of the amino acid sequences of the VH portions of the hybridoma clones 6-1, 6-2 and 56P-1 and the corresponding amino acid sequences of the IgM variable region clone J00529.1 and the IgM constant region clone V00827.1.
Fig. 12 is a drawing showing alignment of the cDNA sequences of the VL portions of the K chains of the hybridoma clones 35-4, 6-1, 6-2 and 56P-1 and the corresponding cDNA sequence of the K chain clone BC088255.1.
Fig. 13 is a drawing showing alignment of the amino acid sequences of the VL portions of the K chains of the hybridoma clones 35-4, 6-1, 6-2 and 56P-1 and the corresponding amino acid sequence of the K chain clone BC088255.1.
Fig. 14 is a drawing showing the in vivo antitumor effect of an antifibrin antibody-SN-38 complex.

### [Description of Embodiments]

The present invention relates to a complex comprising a substance having specific binding affinity for stroma and an antitumor site bound to the substance.

The complex of the present invention, by comprising a substance having specific binding affinity for stroma as a component thereof, possesses specific binding affinity for the stroma. Also, the complex of the present invention is capable of sustained release of an antitumor compound while in a state bound to stroma.

Herein, "interstitium" means connective tissue that fills cell-to-cell gaps in tissue. In the present invention, interstitium particularly means the interstitium of tumor tissue.

Interstitial components include publicly known extracellular matrix constituents. Although the constituents include collagen, elastin, proteoglycan, fibronectin, laminin and the like, and are not particularly limited, as far as they constitute interstitium in tumor tissue; collagen, in particular, is preferable because it is well developed in tumor tissue. Collagen is known to occur in several tens of types, and what type of collagen is expressed predominantly in interstitium differs depending on the kind of tumor; however, generally, type IV collagen is abundant around tumor blood vessels, whereas type I and type III collagens are predominant between tumor cells and tumor blood vessels, so that the collagen is preferably of the type I, type III or type IV, more preferably of the type IV.

In a new aspect, as stroma, a cancer-associated substance can also be used preferably. A cancer-associated substance refers to a substance formed in tumor blood vessels or interstitium in tumor tissue with the growth of tumor cells. Cancer-associated substances include, for example, fibrin.

A substance having specific binding affinity for stroma include an antibody having specific binding affinity for stroma or a binding fragment thereof, a soluble receptor of stroma or a binding fragment thereof, peptides that are compatible with stroma, macromolecular carriers bound with one of the above-described antibodies, soluble receptors, binding fragments, and peptides, and the like. The substance is preferably an antibody having specific binding affinity for stroma, a binding fragment thereof, or a peptide. Here, "specific" means the capability of distinguishing a particular stroma from the components other than the stroma.

Herein, antibodies include, but are not limited to, natural antibodies such as polyclonal antibodies and monoclonal antibodies (mAb) ; chimeric antibodies, humanized antibodies and single-chain antibodies that can be produced using gene recombination technology; and human antibodies that can be produced using human antibody-producing transgenic animals and the like. Antibodies modified with PEG and the like are also encompassed in the antibodies used in the present invention. Preferably, the antibody is a monoclonal antibody, a humanized antibody or a human antibody. The class of antibody is not particularly limited, and includes antibodies of any isotype, such as IgG, IgM, IgA, IgD or IgE. The type is preferably IgG or IgM, more preferably IgG.

A binding fragment of an antibody means a portion of the above-described antibody having specific binding affinity for stroma. Binding fragments of an antibody include F(ab')₂, Fab', Fab, Fv (variable fragment of antibody), sFv, dsFv (disulphide stabilized Fv), sdAb (single domain antibody), antibody fragments prepared using a Fab expression library and the like (Exp. Opin. Ther. Patents, Vol.6, No.5, p.441-456, 1996).

A monoclonal antibody for the present invention can be prepared by a method known per se, and can be prepared by, for example, using the hybridoma method [Nature, vol.256, p.495 (1975)] or using the recombinant DNA method (Cabilly et al., USP 4816567).
For example, interstitial collagen, along with a commercially available adjuvant, is subcutaneously or intraperitoneally administered to mice 2 to 4 times; about 3 days after final administration, the spleen or lymph node is collected, and leukocytes are collected. These leukocytes and myeloma cells (for example, NS-1, P3X63Ag8 and the like) are cell-fused to obtain a hybridoma that produces a monoclonal antibody against the interstitial collagen. The cell fusion may be by the PEG (polyethylene glycol) method [J. Immunol. Methods, 81(2): 223-228 (1985)] or the voltage pulse method [Hybridoma, 7(6): 627-633 (1988)]. A hybridoma that produces a desired monoclonal antibody can be selected by detecting an antibody that binds specifically to an antigen in the culture supernatant using a well-known method of EIA or RIA and the like. Cultivation of a hybridoma that produces a monoclonal antibody can be achieved in vitro, or in vivo in the ascites fluid and the like of a mouse or a rat, preferably a mouse, and the antibody can be acquired from the culture supernatant of the hybridoma or the ascites fluid of the animal.

As a monoclonal antibody for the present invention, a full-length antibody (an antibody as a whole), an antibody fragment (an antibody fragment, for example, Fab', F(ab')₂, scFv (single-chain antibody) and the like), a derivatized antibody or a modified antibody and the like can be used, and a full-length antibody is preferable.

A chimeric antibody can be obtained by joining a DNA that encodes the V region of a monoclonal antibody obtained as described above to a DNA that encodes the C region of a human antibody, integrating this into an expression vector, and introducing the vector into a host to allow it to produce the antibody. Using this known method, a chimeric antibody useful in the present invention can be obtained.

A humanized antibody is also referred to as a reshaped human antibody, and this is prepared by transferring the complementarity determining region (CDR) of an antibody of a non-human mammal, for example, a mouse, to the complementarity determining region of a human antibody; a common technique of gene recombination thereof is known (see European Patent Application Publication No. EP 125023, WO 96/02576).

Specifically, a DNA sequence designed to join the CDR of a mouse antibody and the framework region (FR) of a human antibody is synthesized by a PCR method using several oligonucleotides prepared to have a portion that overlaps the terminal regions of both the CDR and FR as a primer (see a method described in WO 98/13388).

As the framework region of the human antibody joined via the CDR, one that forms an antigen binding portion having a good complementarity determining region is selected. As required, an amino acid in the framework region in the variable region of the antibody may be replaced in a way such that the complementarity determining region of the reshaped human antibody will form an appropriate antigen-binding portion (Sato, K. et al., Cancer Res. (1993) 53, 851-856).

As the C regions of the chimeric antibody and the humanized antibody, those of a human antibody are used; for example, Cγ1, Cγ2, Cγ3, and Cγ4 can be used for H chains, and CK and Cλ for L chains. To improve the stability of the antibody or production thereof, the C region of the human antibody may be modified.

The chimeric antibody consists of the variable region of an antibody derived from a non-human mammal and a constant region derived from a human antibody. Meanwhile, the humanized antibody consists of the complementarity determining region of an antibody derived from a non-human mammal and the framework region and C region derived from a human antibody. Because the humanized antibody has reduced antigenicity in the human body, it is useful as an antibody for use in the present invention.

To improve the stability in living organisms, the substance having specific binding affinity for stroma may have been modified. For example, by modifying the substance with PEG to eliminate the charge, it is possible to protect the substance against phagocytosis by macrophages and the like.

Regarding antitumor compounds that can be used in the present invention, any compound possessing an activity to kill tumor cells in a living organism, such as an anticancer agent, a small-molecule target agent, or a radionuclide, can be used without limitations, including antitumor compounds being used in clinical practices and clinical studies and antitumor compounds that will be developed in the future. Preferably, a compound that time-dependently exhibits an antitumor effect is used.

Herein, an antitumor site means a functional structure possessing antitumor activity. Antitumor sites include antitumor compounds, functional structures capable of sustained release of an antitumor compound, and the like.

When using an antitumor compound as the antitumor site in the complex of the present invention, the molecular weight of the antitumor compound is not particularly limited; however, it is preferable that the molecular weight be so low that after the complex of the present invention is delivered to interstitium in tumor tissue, the antitumor compound is capable of getting released from the complex at the site, moving in the tumor tissue to spread over the entire tumor tissue, and reaching tumor cells. As such, the molecular weight is, for example, 15,000 Da or less, preferably 10,000 Da or less, more preferably 500 Da or less. Also, the molecular weight of the antitumor compound is, for example, 100 Da or more, preferably 300 Da or more. Therefore, a preferred range of the molecular weight of the antitumor compound is 300 to 500 Da.

The antitumor compound is preferably a compound having a hydroxyl group, a carboxyl group or an amino group so as to facilitate the binding of the substance having specific binding affinity for stroma or linker (described below).

It is preferable that the antitumor compound have a weakened antitumor activity (toxicity), that is, in a prodrug state, while in the state bound to the substance having specific binding affinity for stroma or linker, than in the non-bound state.

It is thought that the complex of the present invention, when administered to a living organism, is delivered to interstitium in tumor tissue, stays there, and allows the antitumor compound to be sustainably released from the complex for a long period. Therefore, by using a compound having a time-dependent antitumor effect as the antitumor compound, a higher antitumor effect is expectable. Here, "time-dependence of antitumor effect" means that as the time of persistent exposure to tumor cells increases, the antitumor effect intensifies.

Antitumor compounds that can be used in the present invention include, but are not limited to, for example, alkylating agents such as SN-38 (10-hydroxy-7-ethylcamptothecin), adriamycin, taxol, 5-fluorouracil, nimustine, and ranimustine, metabolism antagonists such as gemcitabine and hydroxycarbamide, plant alkaloids such as etoposide and vincristine, anticancer antibiotics such as mitomycin and bleomycin, platinum preparations such as cisplatin, molecular target agents such as sorafenib and erlotinib, methotrexate, cytosine arabinoside, 6-thioguanine, 6-mercaptopurine, cyclophosphamide, ifosfamide, busulfan and the like. SN-38, in particular, is suitable for use in the present invention because it is unlikely to undergo degradation in the blood.

Of the above-described compounds, time-dependent antitumor compounds include SN-38, taxol, vincristine, methotrexate, cytosine arabinoside, 6-thioguanine, 6-mercaptopurine and the like.

Concentration-dependent antitumor compounds are also preferably used in the present invention from the viewpoint of exposure of a high concentration of an antitumor agent to tumor cells present in tumor tissue. Here, "a concentration-dependent antitumor compound" means an antitumor compound whose cytocidal effect is influenced by the exposure of a higher concentration of an antitumor agent, rather than by time. Of the above-described compounds, 5-fluorouracil, cyclophosphamide, ifosfamide, busulfan and the like can be mentioned.

In the complex of the present invention, when a functional structure capable of sustained release of an antitumor compound is used as the antitumor site, the same as with the use of an antitumor compound as it is as the antitumor site can be used as the antitumor compound.

Examples of the functional structures capable of sustained release of an antitumor compound include liposomes, micelles and the like that contain the antitumor compound.

Examples of the liposome is a vesicle made of lipid bilayer having an aqueous inside. Liposomes include multilayer liposomes, which comprise a number of lipid bilayers in an onion-like form, and monolayer liposomes. The lipid that constitutes the liposome is normally a phospholipid. Phospholipids include phosphatidylcholines such as lecithin and rhizolecithin; acidic phospholipids such as phosphatidylserine, phosphatidylglycerol, phosphatidylinositol, and phosphatidylic acid, or phospholipids wherein these acyl groups have been replaced with a lauroyl group, myristoyl group, oleoyl group or the like, phosphatidylethanolamine, sphingophospholipids such as sphingomyelin and the like. Cholesterol and the like may be added. A liposome containing an antitumor compound can be produced by, for example, suspending a thin film of purified phospholipid in a solution containing the antitumor compound, and performing sonication and the like. A method of producing a liposome containing an antitumor compound is obvious in the technical field of the art. For the method of production, refer to, for example, Annals of Oncology, vol.15, pp.517-525, 2004; Cancer Science, vol.95, pp.608-613, 2004 and the like.

A micelle refers to an aggregate formed as a result of self-association of a solute when its concentration has reached a certain concentration in a solution. A micelle containing an antitumor compound can be obtained by, for example, dissolving a block copolymer and the antitumor compound in an organic solvent (for example, CHCl₃), and evaporating the solvent, and then adding an aqueous solvent, and subjecting the mixture to sonication. Alternatively, the same can be obtained by chemically covalently binding the antitumor compound to the hydrophobic polymer moiety of a block copolymer, and allowing the fusion molecule obtained to self-associate in an aqueous solvent. Here, a block copolymer is a polymer prepared by binding mutually incompatible polymer chains at ends thereof. Examples of the block copolymers that are useful in the present invention include, but are not limited to, polyethylene glycol-poly (glutamic acid) block copolymers, polyethylene glycol-poly (aspartic acid) block copolymers and the like. The antitumor compound contained in the micelle is preferably insoluble or sparingly soluble in water. A method of producing a micelle containing an antitumor compound is obvious in the technical field of the art. For the method of production, refer to, for example, Cancer Research, vol. 66, pp. 10048-10056, 2006 and the like.

To improve the stability in a living organism, the liposome and micelle may have been modified. For example, by modifying the liposome or micelle with PEG to eliminate the charge, it is possible to protect the substance against phagocytosis by macrophages and the like.

Although the particle diameters of the liposome and micelle are not particularly limited, it is preferable that the diameters be set to allow the complex of the present invention to exhibit an EPR effect. As such, the particle diameter is 100 to 450 nm for the liposome and 10 to 80 nm for the micelle. Herein, the particle diameters of the liposome and micelle mean median diameters as measured in PBS at 25°C by the dynamic light scattering method using Particle Sizer NICOMP 380ZLS (Particle Sizing Systems).

In the complex of the present invention, the substance having specific binding affinity for stroma and the antitumor site are bound together directly or indirectly. The bond is normally a covalent bond. "An indirect bond" means a bond via a linker.

In the complex of the present invention, the antitumor site is preferably bound to the substance having specific binding affinity for stroma via a linker. As a result of binding the two molecules via the linker (particularly PEG), the complex exhibits the effect of the present invention in weakening the antigenicity of the substance having the specific binding affinity for stroma.

A technique for joining an antitumor site and a substance having specific binding affinity for stroma via a linker is obvious in the technical field of the art. For ordinary techniques concerning linkers, refer to Hermanson, G.T. (1996). Bioconjugate Techniques, Academic Press; Harris, J.M. and Zalipsky, S., Eds (1997). Poly(ethylene glycol), Chemistry and Biological Applications, ACS Symposium Series; Veronese, F. and Harris, J.M., Eds. (2002). Peptide and protein PEGylation. Advanced Drug Delivery Review 54(4) and the like.

A linker means a divalent or higher (preferably divalent) group that joins two compounds. Examples of the linker that can be used in the present invention include, but are not limited to, a polyalkylene glycol linker, an alkylene group, a peptide, a glycochain, another high molecular carrier and the like. The alkylene moiety of the alkylene glycol that is a constituent unit of the polyalkylene glycol linker normally has 1 to 3,000 carbon atoms, preferably 2 to 1,000 carbon atoms, more preferably 2 to 100 carbon atoms. The molecular weight of the polyalkylene glycol linker is normally 30 to 50,000 Da, preferably 500 to 30,000 Da. The polyalkylene glycol linker is preferably a polyethylene glycol linker. The alkylene group may be linear or branched. The alkylene group normally has 2 to 3,500 carbon atoms, preferably 100 to 2,000 carbon atoms, more preferably 500 to 1,000 carbon atoms.
Those skilled in the art are able to adjust as appropriate the molecular weight of the linker on the basis of the relation described below to the molecular weight of the complex of the present invention and the like.

Linkers include linear linkers (divalent linkers) and branched linkers (trivalent or higher linkers). A linear linker has at one end thereof a portion where it is bound to a substance having specific binding affinity for stroma, and at the other end a portion where it is bound to an antitumor compound. A branched linker normally has at one end thereof a portion where it binds to a substance having specific binding affinity for stroma, to which portion a branching portion joins, to each branch of which branching portion a linear linker (polyalkylene glycol chain, alkylene chain, peptide chain, glycochain and the like) joins, and at the tip of the linker a portion where it binds to an antitumor compound.

The bond between the substance having specific binding affinity for stroma and the linker is a covalent bond or a non-covalent bond (ionic bond, hydrophobic bond and the like), and is preferably a covalent bond. The bond is preferably in a mode such that when administered to a tumor patient, the complex of the present invention is unlikely to undergo cleavage in the blood and is unlikely to undergo cleavage even after being delivered to the interstitium in tumor tissue and binding to the portion. Such bonds include, but are not limited to, a bond between a maleimide group and a thiol group, a bond obtained by reacting a halo ester and a thiol, an amide bond between a carboxyl group and an amino group, a disulfide bond between a thiol group and a thiol group, a Schiff base formed by an amino group and an aldehyde group, a thioester bond between a thiol group and a carboxylic acid, an ester bond between a hydroxyl group and a carboxyl group, a bond formed by an amino group and a squaric acid derivative (for example, dimethylsquaric acid), a bond between a dienyl aldehyde group and an amino group and the like. More specific modes of the bond include a bond between a maleimide group provided at one end of the linker and a thiol group contained in the cysteine residue in the substance having specific binding affinity for stroma, a dehydration-substitution bond between a succinimide group provided at one end of the linker and an amino group contained in the lysine residue in the substance having specific binding affinity for stroma (see, for example, WO 2008/096760), a dehydration condensation bond between an amino group provided at one end of the linker and a carboxylic acid contained in the aspartic acid or glutamic acid in the substance having specific binding affinity for stroma (for example, use of WSCDI) and the like. It is also possible to achieve a bond as a pyridine derivative by a pericyclic reaction between an amino group of the substance having specific binding affinity for stroma and a dienylaldehyde group provided at one end of the linker. When the substance having specific binding affinity for stroma has a cysteine residue at the N-terminus (for example, when a cysteine residue is introduced at the N-terminus by gene alteration), it is possible to join an amino group of the cysteine residue and a linker of the thio ester type via an amide bond (see, for example, Angew. Chem. Int. Ed. Engl. 1997, 36, No.10, pp.1069-1071). Synthesis using intein (protein splicing or protein intron) is also likely.

Examples of specific modes of the bond between a substance having specific binding affinity for stroma and a linker are shown below.

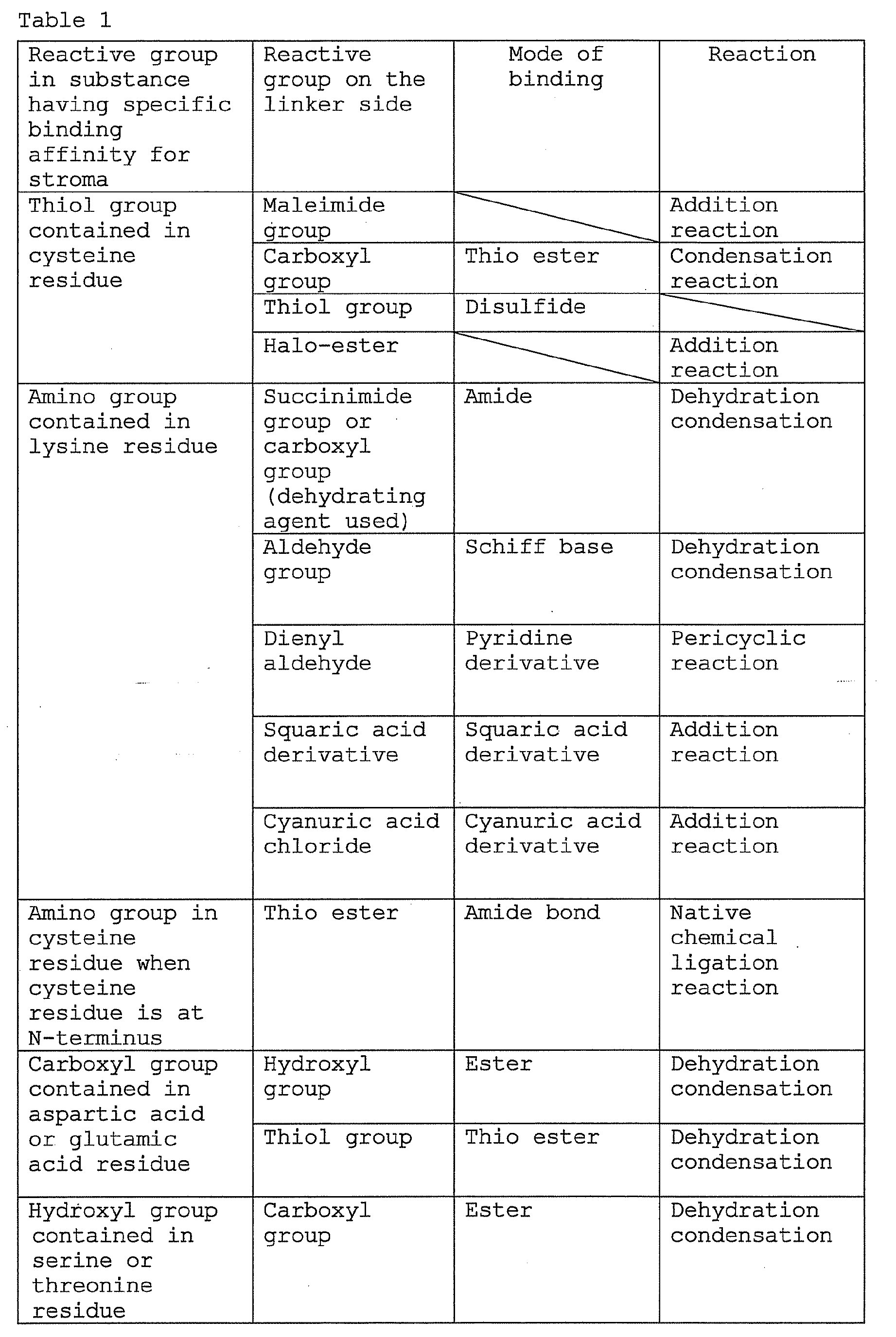

The bond between the substance having specific binding affinity for stroma or linker and the antitumor site is a covalent bond or a non-covalent bond (ionic bond, hydrophobic bond and the like), and is preferably a covalent bond. In particular, when using an antitumor compound as the antitumor site, it is preferable that the bond be in a mode wherein when administered to a tumor patient, the complex of the present invention is unlikely to undergo cleavage in the blood, but after being delivered to tumor interstitium and bound to the site, the antitumor site can be sustainably released from the complex. From this viewpoint, the bond between the substance having specific binding affinity for stroma or linker and the antitumor site is preferably an ester bond or a carbamate bond, more preferably an ester bond, but these are not to be construed as limiting. A carbonate bond, a thiocarbamate bond and the like are also preferable. In case of an ester bond, it is expected that the bond is hydrolyzed by carboxyl esterase in the tumor tissue, or non-enzymatically, to allow the antitumor site to be released sustainably. In case of a carbamate bond, it is expected that the antibody complex is endocytosed as it is in cells and then cleaved by carboxyl esterase in the cells to allow the antitumor site to be released sustainably. In case of a carbonate bond, it is expected that the bond is non-enzymatically hydrolyzed to allow the antitumor compound to be released sustainably. In case of a thiocarbamate bond, it is expected that the bond is non-enzymatically hydrolyzed to allow the antitumor compound to be released sustainably.

As the bond between the substance having specific binding affinity for stroma or linker and the antitumor site, a bond via a hydrazone (dehydration condensation product of carbonyl and hydrazine), a thio ester and the like is also preferable.

When using a liposome or micelle containing an antitumor compound as the antitumor site, the antitumor compound can be sustainably released from the liposome or micelle due to the structure thereof. Therefore, the bond between the substance having specific binding affinity for stroma or linker and the liposome or micelle may be in a mode wherein when administered to a tumor patient, the complex of the present invention is unlikely to undergo cleavage both in the blood and in tumor interstitium. Such kinds of bonds include, for example, a bond described in WO 00/64413.

Specific examples of linear linkers include a linker represented by the formula:

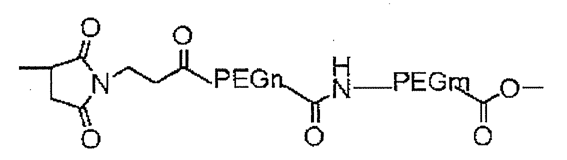

(wherein PEG represents a polyethylene glycol chain, and each of n and m is a number of ethylene glycol units and independently represents an integer of 5 to 100). The linker normally joins to the substance having specific binding affinity for stroma at an end having a succinimidyl group, and joins to the antitumor compound at the other end.

Furthermore, specific examples of linear linkers include a linker represented by the formula:

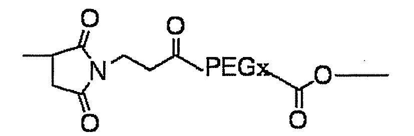

(wherein PEG represents a polyethylene glycol chain, and x is a number of ethylene glycol units and represents an integer of 5 to 100). The linker normally joins to the substance having specific binding affinity for stroma at an end having a succinimidyl group, and joins to the antitumor compound at the other end.

Specific examples of branched linkers include a linker represented by the formula:

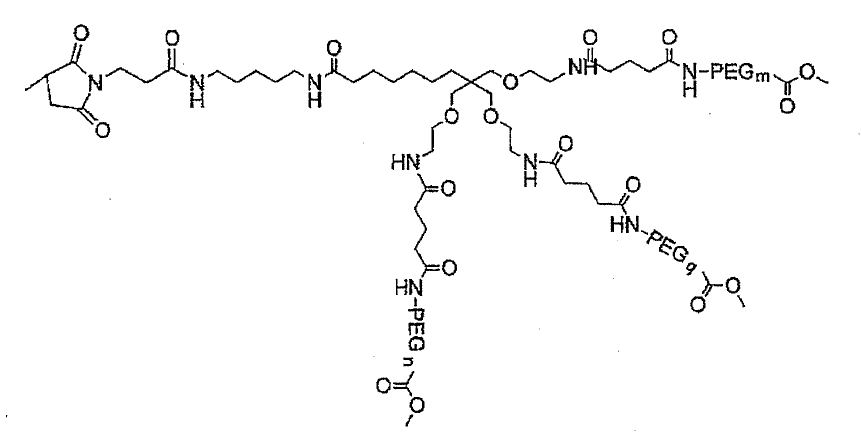

(wherein PEG represents a polyethylene glycol chain, and each of n, m and q is a number of ethylene glycol units and independently represents an integer of 5 to 100). The linker normally joins to the substance having specific binding affinity for stroma at an end having a succinimidyl group, and joins to the antitumor compound at a plurality of other ends. This branched linker is available from, for example, Pierce Company.

When using an antitumor compound as the antitumor site as it is in the complex of the present invention, the number of molecules of the antitumor compound bound to each molecule of the substance having specific binding affinity for stroma is theoretically not particularly limited; however, from the viewpoint of the stability of the complex, the ease of production and the like, the number is normally 1 to 10, preferably 1 to 8.
Although the molar ratio of the linker moiety and antitumor compound moiety in the complex is normally 1:1, the antitumor compound may account for several moles relative to 1 mol of the linker moiety.

When using a functional structure capable of sustained release of an antitumor compound (e.g., liposomes, micelles) as the antitumor site in the complex of the present invention, the ratio of the substance having specific binding affinity for stroma that binds to the functional structure is theoretically not particularly limited; however, from the viewpoint of the stability of the complex, the ease of production and the like, the number is determined as with the complexes described in Y. Matsumura et al. Phase I and pharmacokinetic study of MCC-465, a doxorubicin (DXR) encapsulated in PEG immunoliposome, in patients with metastatic stomach cancer. Annals of Oncology. 2004: 15: 517-525, F. Koizumi et al. Novel SN-38-Incorporating Polymeric Micelles, NK012, Eradicate Vascular Endothelial Growth Factor-Secreting Bulky Tumors. Cancer Res. 2006: 66 (20): 10048-10056, T. Hamaguchi et al. Antitumor effect of MCC-465, pegylated liposomal doxorubicin tagged with newly developed monoclonal antibody GAH, in colorectal cancer xenografts. Cancer Sci. 2004: 95: 608-613.
Although the molar ratio of the linker moiety and the functional structure moiety in the complex is normally 1:1, the functional structure may account for several moles relative to 1 mole of the linker moiety.

Regarding angiological characteristics of tumors, it has been reported that accentuated tumor vascular permeability has occurred to allow macromolecular substances, which are unlikely to leak from normal blood vessels, to readily leak from tumor blood vessels, and that due to a lack of lymphangiogenesis over neovascularization, there is an EPR effect (enhanced permeation and retention effect) wherein macromolecular substances that have once leaked from blood vessels in tumor tissue cannot be drained to lymph vessels and stay long in the tumor tissue. Although the size (molecular weight and the like) of the complex of the present invention is not particularly limited, it is preferable that the size be such one that allows the complex to exhibit an EPR effect.

When using an antitumor compound as the antitumor site as it is, the range of the molecular weight of the complex in which the complex can exhibit an EPR effect is, for example, 50,000 Da or more, preferably 70,000 Da or more, but this is not to be construed as limiting, as far as an EPR effect can be exhibited. If the molecular weight is under 50,000 Da, the molecule unavoidably gets secreted from the kidney into urine, so that the risk of weakening of the EPR effect increases. Meanwhile, if the molecular weight is too high, the risk that the molecule would be recognized as a foreign matter and phagocytosed by macrophages increases; therefore, the molecular weight of the complex of the present invention is normally 200,000 Da or less. Therefore, the molecular weight of the complex of the present invention is, for example, preferably in the range of 100,000 to 180,000 Da, more preferably 120,000 to 160,000 Da, still more preferably 150,000 Da. Generally, the molecular weight of a protein that suitably exhibits a good EPR effect is known to be 70,000 to 200,000 Da.

When using a functional structure capable of sustained release of an antitumor compound (e.g., liposomes, micelles) as the antitumor site, the particle diameter of the complex capable of exhibiting an EPR effect differs depending on the choice of functional structure. For example, when using a liposome, the particle diameter of the complex capable of exhibiting an EPR effect is normally 100 to 450 nm. When using a micelle, the particle diameter of the complex capable of exhibiting an EPR effect is normally 10 to 80 nm. Herein, the particle diameter of the complex means a median diameter as measured in PBS at 25°C using the dynamic light scattering method with the Particle Sizer NICOMP 380ZLS (Particle Sizing Systems).

Although the molecular weight (or particle diameter) of each of the substance having specific binding affinity for stroma, linker, antitumor compound or functional structure capable of sustained release of the antitumor compound is not particular limited, it is preferable that these molecular weights be set to allow the complex of the present invention to exhibit an EPR effect. The molecular weight of the substance having specific binding affinity for stroma is, for example, 50,000 Da or more, preferably 70,000 Da or more, when the substance is a protein; however, this is not to be construed as limiting, as far as an EPR effect can be exhibited. If the molecular weight is under 50,000 Da, the molecule gets excreted from the kidney into urine, resulting in an increased risk that the EPR effect weakens. For example, F (ab) whose molecular weight is 25,000 Da is readily excreted from the kidney, so that the accumulation in tumors is sometimes limited. Meanwhile, if the molecular weight is too high, the risk that the molecule is recognized as a foreign matter and phagocytosed by macrophages increases; for example, an IgM antibody whose molecular weight is 900,000 Da is so large that it is unlikely to leak from tumor blood vessels and likely to be captured by the reticuloendothelial system in the liver and the like. The molecular weight of the substance having specific binding affinity for stroma is normally 200,000 Da or less, when the substance is a protein. It is generally known that the molecular weight of a protein that suitably exhibits a good EPR effect is 70,000 to 200,000 Da (non-patent documents 13 and 17). From this viewpoint as well, the antibody for use in the present invention is preferably IgG (molecular weight about 150,000 Da).

The complex of the present invention can be produced by binding a substance having specific binding affinity for stroma to an antitumor site. When using a linker to bind the two, the complex of the present invention can be produced by binding the linker to the antitumor site, and further binding this to the substance having specific binding affinity for stroma. The order of binding the individual moieties is not limited to this order.

An example case is hereinafter explained wherein SN-38 (10-hydroxy-7-ethylcamptothecin) is used as the antitumor site, a polyethylene glycol linker as the linker, and an antibody as the substance having specific binding affinity for stroma; however, even in case of other combinations of the three ingredients, those skilled in the art are able to produce the desired complex of the present invention by altering the reaction conditions as appropriate.

(I) First, a polyethylene glycol having a carboxyl group at one end thereof and an amino group protected by Boc, Fmoc and the like at the other end and SN-38 are dehydrate-condensed to introduce the polyethylene glycol linker into the hydroxyl group of SN-38.

(II) By mixing a polyethylene glycol having a succinimide group at one end thereof and a maleimide group at the other end and the product (I) to react the succinimide group and the amino group of the product (I), the maleimide group is introduced into the polyethylene glycol linker.

(III) By mixing the product (II) and an antibody to react the maleimide group in the product (II) and the thiol group in the antibody and bind the product (II) and the antibody, the complex of the present invention is obtained.

Because the complex of the present invention is characterized in that it binds to the interstitium in tumor tissue and stays in the tumor tissue for a long period to continue to exhibit an antitumor effect for a long period, tumors in a mammal can be prevented or treated by administering an effective amount of the complex of the present invention to the mammal. Furthermore, the complex of the present invention is also capable of exhibiting an antitumor effect for a long period by staying in tumor tissue for a long period to inhibit the formation of blood vessels that feed the tumor in the tumor boundary region. Accordingly, the present invention provides a tumor prophylactic or therapeutic agent and a tumor blood vessel inhibitor that comprises the above-described the complex of the present invention (hereinafter, these are also referred to as an agent of the present invention).

Although the kind of tumor is not particularly limited, from the viewpoint of allowing the above-described features to be manifested to the fullest, the tumor is a solid cancer, preferably a solid cancer having interstitium. Kinds of solid cancer include, but are not limited to, osteosarcoma, esophageal cancer, lung cancer, liver cancer, gastric cancer, pancreatic cancer, colorectal cancer, rectal cancer, colic cancer, ureteral tumor, brain tumor, gallbladder cancer, cholangioma, bile duct cancer, renal cancer, breast cancer, urinary bladder cancer, ovarian cancer, uterocervical cancer, prostatic cancer, thyroid cancer, testicle tumor, Kaposi's sarcoma, maxillary cancer, tongue cancer, lip cancer, oral cancer, laryngeal cancer, pharyngeal cancer, myosarcoma, skin cancer and the like. In particular, the complex of the present invention is beneficial in the prevention and treatment of interstitium-rich tumors (for example, intractable cancers such as pancreatic cancer, gastric cancer (scirrhous gastric cancer), colorectal cancer, and lung cancer) and the inhibition of the formation of blood vessels that feed tumors.

An agent of the present invention can be applied to tumors in an optionally chosen mammal. Useful mammalian species include laboratory animals such as mice, rats, hamsters, guinea pigs, and other rodents, and rabbits; domestic animals such as swines, bovines, goat, horses, sheep, and minks; companion animals such as dogs and cats; and primates such as humans, monkeys, rhesus monkeys, marmosets, orangutans, and chimpanzees. Because the tumor tissues of primates such as humans are generally richer in interstitium than the tumor tissues of rodents such as mice, an agent of the present invention is beneficial in the prevention and treatment of tumors and the inhibition of the formation of tumor blood vessels in primates, particularly in humans.

An agent of the present invention can be used as an oral preparation or a non-oral preparation alone or after being prepared as a preparation along with pharmacologically acceptable additives such as carriers, flavoring agents, excipients, antiseptics, suspending agents, solvents, solubilizers, isotonizing agents, swelling agents, disintegrants, lubricants, sweetening agents, and binders according to a routine method.

Binders include gelatin, cornstarch, tragacanth, gum arabic, α-starch, sucrose, methylcellulose, carboxymethylcellulose, carboxymethylcellulose sodium, crystalline cellulose, sucrose, D-mannitol, trehalose, dextrin, pullulan, hydroxypropylcellulose, hydroxypropylmethylcellulose, polyvinylpyrrolidone and the like.
Excipients include lactose, sucrose, D-mannitol, D-sorbitol, starch, α-starch, dextrin, crystalline cellulose, low-substitution hydroxypropylcellulose, carboxymethylcepallulose sodium, gum arabic, pullulan, soft silicic anhydride, synthetic aluminum silicate, magnesium metasilicoaluminate, xylitol, sorbitol, erythritol and the like.
Lubricants include magnesium stearate, calcium stearate, talc, colloidal silica, polyethylene glycol and the like.
Sweetening agents include saccharin sodium, dipotassium glycyrrhizinate, aspartame, stevia and the like.
Flavoring agents include peppermint, agamont oil and cherry.
Antiseptics include para-oxybenzoic acid esters, chlorobutanol, benzyl alcohol, phenethyl alcohol, dehydroacetic acid, and sorbic acid.
Disintegrants include lactose, sucrose, starch, carboxymethylcellulose, carboxymethylcellulose calcium, crosslinked carmellose sodium, carboxymethyl starch sodium, low-substitution hydroxypropylcellulose, soft silicic anhydride, calcium carbonate and the like.
Suspending agents include, for example, surfactants such as stearyltriethanolamine, sodium lauryl sulfate, lauryl aminopropionic acid, lecithin, benzalkonium chloride, benzethonium chloride, and monostearic glycerol; hydrophilic polymers, for example, polyvinyl alcohol, polyvinylpyrrolidone, carboxymethylcellulose sodium, methylcellulose, hydroxymethylcellulose, hydroxyethylcellulose, hydroxypropylcellulose and the like; polysorbates, polyoxyethylene hardened castor oil and the like.
Examples of suitable solvents include water for injection, physiological saline, Ringer's solution, alcohol, propylene glycol, polyethylene glycol, sesame oil, corn oil, olive oil, cottonseed oil and the like.
Examples of suitable solubilizers include polyethylene glycol, propylene glycol, D-mannitol, trehalose, benzyl benzoate, ethanol, tris-aminomethane, cholesterol, triethanolamine, sodium carbonate, sodium citrate, sodium salicylate, sodium acetate and the like.
Examples of suitable isotonizing agents include sodium chloride, glycerin, D-mannitol, D-sorbitol, glucose, xylitol, fructose and the like.

Also, an agent of the present invention may be formulated with, for example, a buffer, a soothing agent, a stabilizer, a preservative, an antioxidant, a coloring agent and the like.
Buffers include buffer solutions of phosphates, acetates, carbonates, citrates and the like, and the like.
Soothing agents include propylene glycol, lidocaine hydrochloride, benzyl alcohol, benzalkonium chloride, procaine hydrochloride and the like.
Stabilizers include human serum albumin, polyethylene glycol and the like.
Preservatives include benzyl alcohol, phenol and the like.
Antioxidants include sulfites, ascorbates and the like.
Examples of suitable coloring agents include water-soluble food tar colors (e.g., food colors such as Food Red Nos. 2 and 3, Food Yellow Nos. 4 and 5, and Food Blue Nos. 1 and 2), insoluble lake pigments (e.g., aluminum salts of the aforementioned water-soluble food tar colors), natural pigments (e.g., β-carotene, chlorophyll, red iron oxide) and the like.

Regarding the route of administration, it is desirable to use the therapeutically most effective one; an agent of the present invention can be administered in, for example, oral preparations, injections or transdermal preparations. Oral preparations include tablets (including sublingual tablets and oral disintegrants), capsules (including soft capsules and microcapsules), powders, granules, troches, syrups, emulsions, suspensions and the like. Injections include intradermal injections, subcutaneous injections, intravenous injections, intramuscular injections, intraspinal injections, epidural injections, topical injections and the like. Transdermal preparations include patches, ointments, dusting powders and the like. These preparations may be release-controlled preparations such as quick-release preparations or sustained-release preparations (e.g., sustained-release microcapsules).

An agent of the present invention is suitably formulated as an injection. A sterile composition for injections can be formulated according to ordinary procedures of preparation making such as by dissolving or suspending an active substance in a vehicle (aqueous solutions for injection; naturally produced vegetable oils such as sesame oil and coconut oil, and the like). Aqueous solutions for injection that can be used include, for example, physiological saline, isotonic solutions containing glucose or another auxiliary drug (for example, D-sorbitol, D-mannitol, sodium chloride and the like) and the like, which may be used in combination with appropriate solubilizers, for example, alcohols (e.g., ethanol), polyalcohols (e.g., propylene glycol, polyethylene glycol), nonionic surfactants (e.g., polysorbate 80™, HCO-50) and the like. Useful oily solutions include, for example, sesame oil, soybean oil and the like, which may be used in combination with a solubilizer such as benzyl benzoate or benzyl alcohol. These injections can be encapsulated in containers such as ampoules and vials for unit dosage or a plurality of dosages. It is also possible to freeze-dry an active ingredient and a pharmaceutically acceptable carrier, and store the preparation in a state that may be dissolved or suspended in an appropriate sterile vehicle just before use.

The content amount of the complex of the present invention in an agent of the present invention differs depending on the form of preparation, and is normally about 0.1 to 99.9% by weight, preferably about 1 to 99% by weight, more preferably about 10 to 90% by weight, relative to the preparation as a whole.

The dosage of an agent of the present invention can be determined as appropriate in view of the species of the recipient of administration, method of administration, choice of antitumor agent, kind of tumor cell, site and the like; when administered by intravenous injection for human solid cancer, the amount of the complex of the present invention per kg of body weight is preferably 5 to 500 mg, more preferably 10 to 500 mg, still more preferably 10 to 300 mg. These effective amounts can be administered at one time or in several divided portions.

In another mode of embodiment, the present invention provides a complex consisting of a substance having specific binding affinity for stroma and a marker compound bound to the substance via a linker.
Marker compounds include, but are not limited to, radioisotopes, fluorescent substances or enzymes and the like.
Specifically, radioisotopes such as ³H, ¹⁴C, ¹²⁵I and ¹³¹I, fluorescent substances such as green fluorescent protein (GFP), fluorescein isothiocyanate, tetramethylrhodamine isothiocyanate, and Eu³⁺, methylcoumarin-series compounds, enzymes such as peroxidase, alkaline phosphatase, β-D-galactosidase, glucose oxidase, and glucose-hexaphosphate dehydrogenase can be mentioned as marker compounds.
In case of enzymatic labeling, chemical conversion of a detectable substrate compound or composition may be catalyzed.
The definitions of the above-described substance having specific binding affinity for stroma and the linker are as described above.
The mode of binding of the substance having specific binding affinity for stroma and the linker is as described above; the linker and the marker compound can be determined as appropriate as with the above-described mode of binding of the linker and the antitumor compound.
The particle diameter of the complex and the molecular weights of the entire complex, the substance having specific binding affinity for stroma, the linker, and the marker compound are as described above.
The complex of the present invention can be obtained as with the above-described method of production.
Because the complex is capable of staying in the interstitium portion of tumor tissue for a long time, it may be used as a diagnostic reagent for the presence or absence of tumor, size, imaging and the like, as it is, or after being prepared as a preparation along with additives described below.
The present invention also provides a tumor diagnostic method comprising using the above-described complex to a subject.

The content amount of the complex of the present invention in the diagnostic reagent differs depending on the form of preparation, and can be determined as with the content amount of the complex of the present invention in the aforementioned therapeutic agent. The amount of the diagnostic drug of the present invention used can also be determined as with the dosage of the aforementioned therapeutic agent. These can be used at one time or in several divided portions.

The present invention is hereinafter described more specifically by means of the following examples, to which, however, the invention is never limited.

### Examples

### (Reference Example 1)

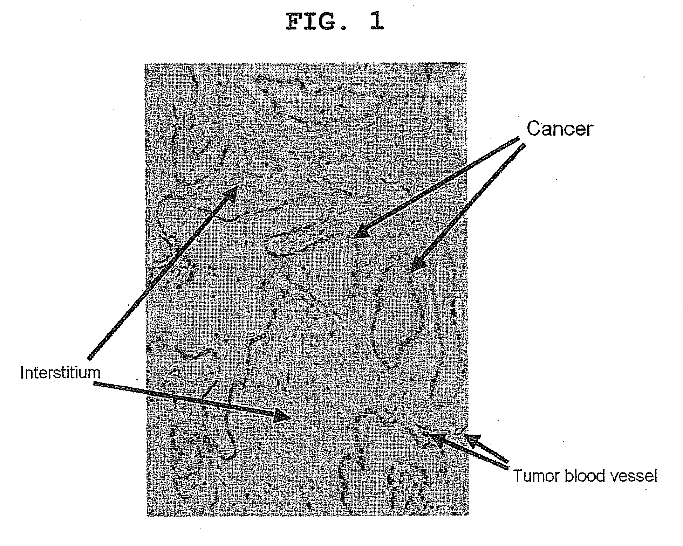

A surgical specimen of human pancreatic cancer is shown in Fig. 1. The specimen was poor in tumor blood vessels, with only some tumor cells being present in the tumor tissue mass, the portion surrounding the blood vessels being filled with interstitium, including collagen.

Meanwhile, the distribution of interstitial collagen in tumors that developed from the human pancreatic cancer line PSN1 or SUIT2 that had been subcutaneously transplanted to nude mice was examined by triple staining of EpCAM with a mouse antihuman EpCAM antibody (B8-4, generated by the National Cancer Center) and an antimouse Alexa488-labeled secondary antibody (Invitorogen), of collagen type 4 with a rat antimouse collagen 4 antibody (1-4, generated by the National Cancer Center) and an anti-rat Alexa555-labeled secondary antibody (Invitrogen), and of the cell nucleus with DAPI (Invitogen), and imaging using the fluorescence microscope BZ9000 (Keyence). Although the degree was not as high as with human pancreatic cancer, abundant collagen was noted in interstitium. More interstitial collagen was noted in SUIT2 than in PSN1.

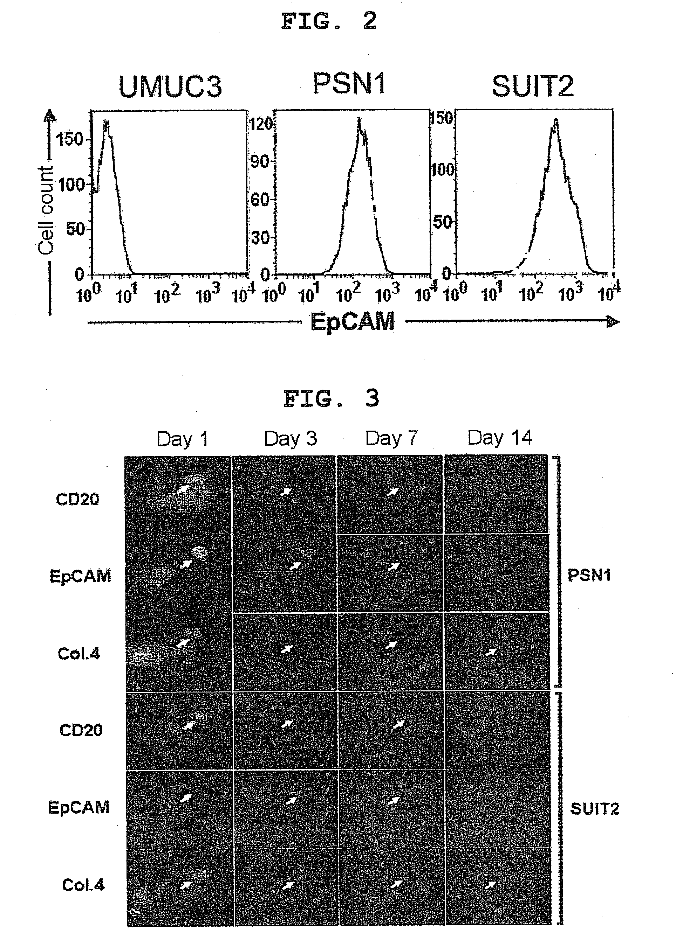

With the urinary bladder cancer cell line UMUC3 for negative control, the pancreatic cancer cell lines PSN1 and SUIT2 were examined for the expression of EpCAM measured using a flow cytometer. Specifically, each cell line was co-stained with a mouse antihuman EpCAM antibody (B8-4) as the primary antibody and an APC-labeled antimouse antibody (Beckton Dickinson and Company) as the secondary antibody, further using PI (propidium iodide, Invitrogen) for removing dead cell measurements, after which the expression was measured using the flow cytometer FACSCaribur (Beckton Dickinson and Company). Analysis was performed using the analytical software Flowjo (Tree Star). Results are shown in Fig. 2. From these results, it is evident that the human pancreatic cancer line SUIT2 is EpCAM (Epithelial Cell Adhesion Molecule)-positive cells.

### (Reference Example 2)

### In vivo imaging of antibody

Using for control an antihuman CD20 antibody (an antibody that does not react with the human pancreatic cancer line SUIT2, Rituximab, Chugai Pharmaceutical Co., Ltd.), which is a monoclonal antibody against human B cells, the accumulation of an antihuman EpCAM antibody (B8-4) and an antimouse type IV collagen antibody (35-4) in tumors was investigated.
PSN1 or SUIT2 was transplanted to the back of each BALB/c nude mouse (female, 6-week-old); 10 days after the transplantation, each antibody (labeled with IRDye800 (Li-Cor)) was administered from a tail vein of the mouse. 1 day, 3 days, 7 days, and 14 days after the administration, the antibody distribution was analyzed using the in vivo imaging apparatus OV110 (Olympus).
Results are shown in Fig. 3. In the figure, CD20 stands for the antihuman CD20 antibody, EpCAM for the antihuman EpCAM antibody, and Col.4 for the antimouse type IV collagen antibody.
The antihuman CD20 antibody did not exhibit an antigen-antibody reaction, but accumulated selectively in tumor tissue due to an EPR effect. However, it disappeared from the tumor tissue earlier than the antimouse type IV collagen antibody. Although the antihuman EpCAM antibody was also found to accumulate in the tumor tissue, it disappeared earlier than the antimouse type IV collagen antibody despite the fact that it is specific for the SUIT2 tumor. The antimouse type IV collagen antibody accumulated in the tumor tissue for a longer period than the two other antibodies despite the presence of the neutralizing antigen mouse type IV collagen in the mouse blood, demonstrating its high accumulation in tumors.

The results above suggested that an antitumor compound can be allowed to stay in tumor tissue for a longer period when using a substance having specific binding affinity for stroma in the tumor tissue for targeting, rather than when using an antibody against a tumor cell surface antigen for targeting.

### (Example 1)

### Production of SN-38-linker-antibody complex

### 1. Binding of polymer and SN-38

The antitumor compound SN-38 was bound to a linker.

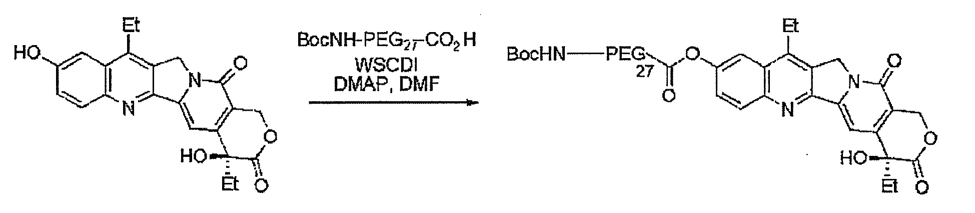

WSCDI (water soluble carbodiimide: 54.8 mg, 0.286 mmol) was added to a DMF solution (1-mL) of 10-hydroxy-7-ethylcamptothecin (102.1 mg, 0.260 mmol), Boc-PEG₂₇-COOH (407.1 mg, 0.286 mmol), and DMAP (15.9 mg, 0.130 mmol) at 0°C. The mixture was stirred at room temperature for 19 hours, and the reaction mixture was purified by gel permeation column chromatography (LH20 CHCl₃:MeOH = 1:1) and silica gel column chromatography (CHCl₃:MeOH = 15:1-9:1) to yield an ester as a colorless oily substance (420.1 mg, 90%).
¹H-NMR δ (CD₃OD) 8.20 (d, J = 9.2 Hz, 1H), 7.99 (s, 1H), 7.66 (m, 2H), 5.60 (d, J = 16.5 Hz, 1H), 5.40 (d, J = 16.5 Hz, 1H), 5.32 (M, 2H), 3.93 (t, J = 6.4 Hz, 2H), 2.96 (t, J = 6.4 Hz, 2H), 1.97 (m, 2H), 1.43 (s, 9H), 1.40 (t, J = 7.6 Hz, 3H), 1,02 (t, J = 7.8 Hz, 3H); ¹³C-NMR (DMSO-d₆) 5164.8, 161.9, 149.2, 148.5, 143.1, 142.7, 141.3, 138.2, 137.7, 137.5, 122.4, 119.5, 118.9, 117.2, 110.7, 106.5, 89.7, 70.4, 64.6, 62.3, 62.0, 62.0, 62.0, 61.9, 61.8, 61.8, 61.7, 61.5, 58.1, 58.0, 57.1, 41.2, 40.1, 31.8, 28.1, 26.3, 19.4, 14.4, 4.9, -1.1.

### 2. Introduction of maleimide group into linker

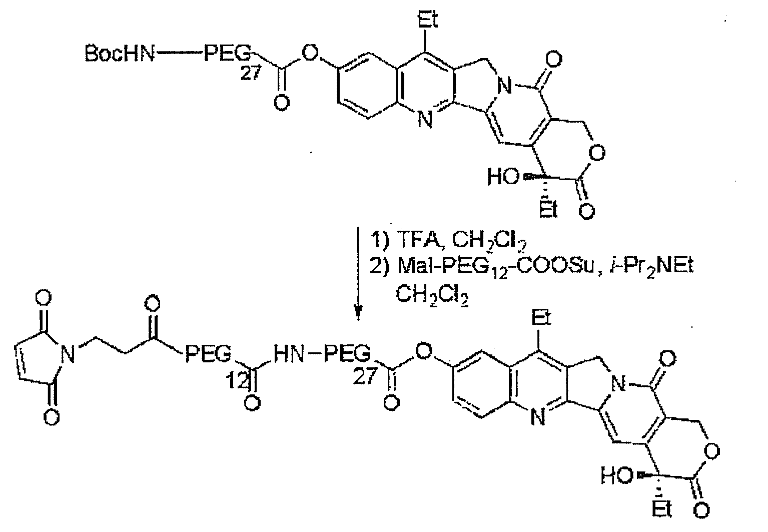

Furthermore, TFA (1 mL) was added to a solution of Boc-PEG₂₇-camptothecin (221.5 mg, 0.123 mmol) in CH₂Cl₂ (10 mL) at room temperature. The mixture was stirred for 1.5 hours, after which the solvent was evaporated off in a vacuum, and toluene was added. After evaporation, the residue was dried in a high vacuum. The residue was dissolved in CH₂Cl₂ (5 mL) and MAL-PEG₁₂-NHS (128.2 mg, 0.148 mmol), and iPr₂NEt (48 µL, 0.246 mmol) was added at 0°C. Thirty minutes later, the mixture was purified by gel permeation column chromatography (LH-20, CHCl₃:CH₃OH = 1:1) and silica gel column chromatography to yield the desired product as a colorless oily substance (276.0 mg, 91%).
¹H-NMR (CD₃OD) δ 8.20 (d, J = 9.2 Hz, 1H) , 8.00 (s, 1H), 7.66-7.64 (m, 2H), 6.83 (s, 2H), 5.60 (d, J = 16.0 Hz, 1H), 5.41 (d, J = 16.0 Hz, 1H), 5.34 (s, 1H), 3.93 (t, J = 6.0 Hz, 2H), 2.97 (t, J = 6.0 Hz, 2H), 2.49-2.45 (m, 4H), 2.00-1.98 (m, 2H), 1.41 (t, J = 7.6 Hz, 3H), 1.02 (t, J = 7.6 Hz, 3H); ¹³C-NMR (DMSO-d₆) δ 172.1, 170.4, 169.8, 169.7, 169.1, 156.5, 151.7, 149.7, 148.9, 146.2, 145.6, 145.0, 134.3, 131.1, 128.4, 126.8, 125.3, 118.8, 115.0, 96.5, 72.3, 69.9, 69.6, 69.5, 69.4, 69.0, 68.9, 66.7, 65.8, 65.1, 49.5, 36.0, 34.8, 34.1, 33.9, 31.6, 30.3, 25.4, 22.3, 13.9, 7.8.

### 3. Binding of SN-38-polymer compound and antibodies

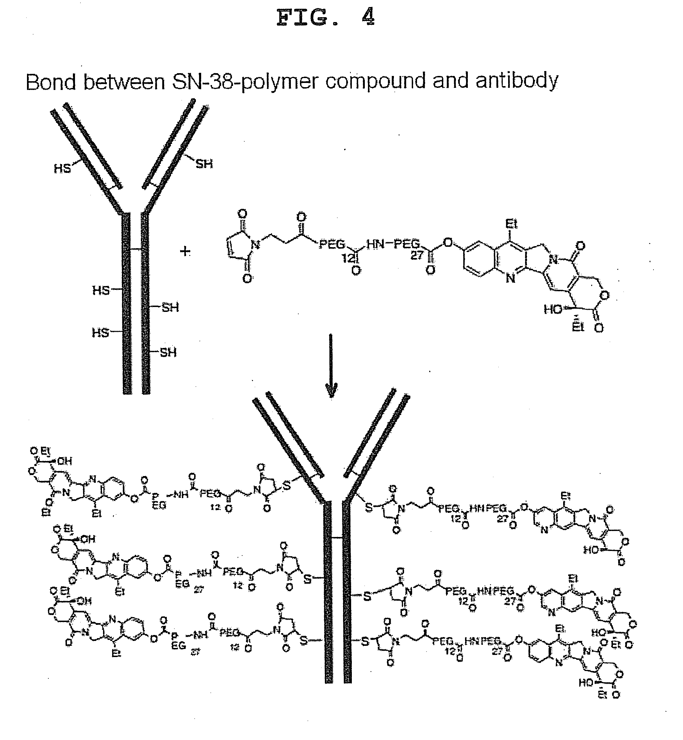

An antihuman EpCAM antibody (B8-4) generated by the National Cancer Center, an antimouse type IV collagen antibody (35-4), and an antihuman CD20 antibody (Rituximab, Chugai Pharmaceutical Co., Ltd.) were prepared to obtain a concentration of 1.0 mg/ml in PBS for each antibody. DTT (dithiothreitol) (Sigma) was added to obtain a final concentration of 10 mM, and allowed to react with each antibody at 37°C for 30 minutes; the reaction reagent was removed by ultrafiltration (Amicon Ultra Centrigugal Filter Devices, Milipore Co). The reaction product obtained was subjected to absorptiometry using a Spectrophotometer (NanoDrop, SCRUM Inc.); the antibody recovery rate was about 80%. Judging from the quantitation results for SH groups by the DNTB (Dinitrothiocyanobenzene, Wako) method, it was thought that 8.7 SH groups per antibody were obtained with the antihuman EpCAM antibody, 7.2 SH groups with the antihuman CD20 antibody, and 7.7 SH groups with the antimouse type IV collagen antibody. Next, the above-described reaction product was dissolved in 100 mM phosphate-buffered solution + 150 mM NaCl + 5 mM EDTA (pH 6.0) so that the protein concentration would be 0.5 mg/ml, and the maleimide compound and each antibody were mixed in a ratio of 4 mol of the maleimide compound to 1 mol of the antibody, after which they were reacted at room temperature for 1 hour and then at 4°C overnight. The reaction reagent was removed by ultrafiltration and replaced with PBS. The amount of protein was measured by the Bradford method (Bio-Rad Protein Assay, 500-0006JA, Bio-Rad). The protein recovery rate was 51% for the EpCAM antibody, 77% for the CD20 antibody, and 51% for the 35-4 antibody, the number of SN-38 units added per antibody being 8.4 for the EpCAM antibody, 6.7 for the CD20 antibody, and 7.2 for the anti-collagen antibody. Calculations were likewise made by the DNTB method. A schematic diagram of a complex obtained is shown in Fig. 4.

### (Example 2)

### In vitro cytocidal effect

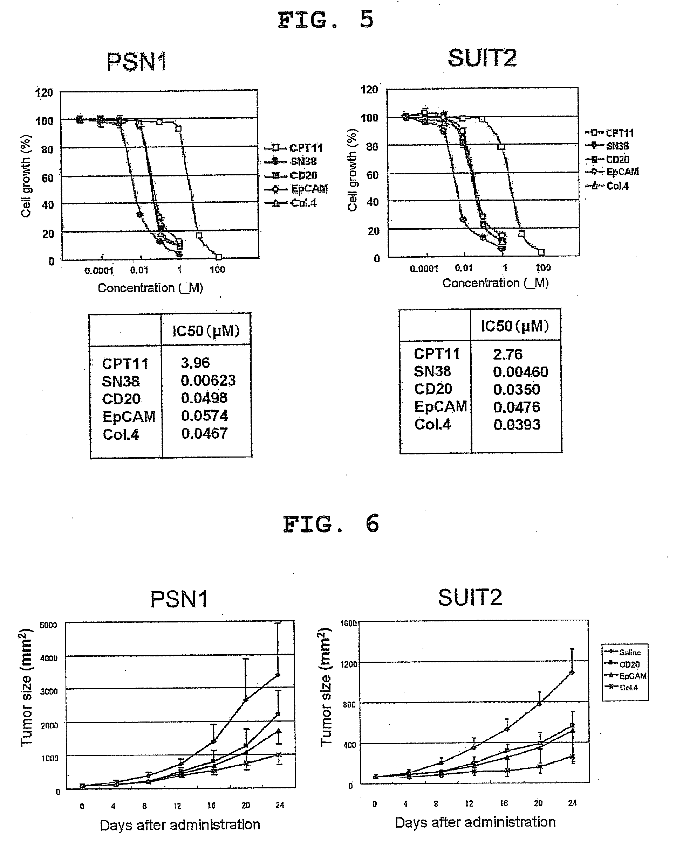

The in vitro cytocidal effects of the antihuman CD20 antibody-SN-38 complex, antihuman EpCAM antibody-SN-38 antibody and antimouse type IV collagen antibody-SN-38 complex prepared in Example 1 were compared with free SN-38 and CPT-11.
3000 PSN1 or SUIT2 cancer cells were seeded to a 96-well cell plate; 24 hours later, each complex was added; 48 hours later, cell counts were measured by the WST-8 method using the Cell Counting Kit-8 (Dojindo). The results are shown in Fig. 5.
As a result, the antitumor effects of the antihuman CD20 antibody-SN-38 complex, the antihuman EpCAM antibody-SN-38 antibody, and the antimouse type IV collagen antibody-SN-38 complex were weaker than that of free SN-38, but they maintained an antitumor effect exceeding that of CPT-11. No difference in antitumor effect was noted among the three kinds of complexes.

### (Example 3)

### In vivo antitumor effect

PSN1 or SUIT2 was subcutaneously transplanted to nude mice; when the tumor diameter reached 6 mm, the antihuman CD20 antibody-SN-38 complex, the antihuman EpCAM antibody-SN-38 antibody or the antimouse type IV collagen antibody-SN-38 complex was intravenously administered (3 mg/kg each, based on SN-38), and the size of tumor mass was monitored. The results are shown in Fig. 6. No difference in antitumor effect was noted among the three kinds of complexes in vitro; however, in vivo, the antimouse type IV collagen antibody-SN-38 complex exhibited the highest antitumor effect.

The results above suggested that a higher antitumor effect can be achieved in vivo when using a substance having specific binding affinity for a component of tumor interstitium for targeting, rather than when using an antibody against a tumor cell surface antigen for targeting.

### (Example 4)

### Action on tumor cells and tumor blood vessels

SUIT2 was subcutaneously transplanted to nude mice; when the tumor diameter reached 6 mm, the antimouse type IV collagen antibody-SN-38 complex was intravenously administered (3 mg/kg each, based on SN-38), and histopathological features were monitored. Details are shown below.

### (Materials and Methods)

### Antibodies/drugs and reagents

A hybridoma that produces an anti-EpCAM antibody (clone B8-4) was obtained from a mouse immunized with a recombinant protein (R&D Systems, Minneapolis, MN, USA). A hybridoma that produces an anti-collagen IV antibody (clone 35-4) was obtained from a rat immunized with a purified protein. Splenocytes obtained from each immunized mouse were fused with myeloma cells (P3X63Ag8.653). As the particular antibody that produces a hybridoma clone, a binding recombinant protein was selected using ELISA. An antihuman CD20 antibody (rituximab) was purchased from Daiichi Sankyo (Tokyo, Japan). For immunohistochemistry, a polyclonal anti-collagen IV antibody (LSL-LB-1403) and a monoclonal anti-CD31 antibody (MEC13.3) were purchased from Cosmo Bio (Tokyo, Japan) and Becton and Dickinson (Franklin Lakes, NJ, USA), respectively. SN-38 and CPT-11 (irinotecan) were purchased from Tokyo Chemical Industry Co., Ltd. (Tokyo, Japan) and Yakult (Tokyo, Japan), respectively.

### Cell line

The human pancreatic cancer cell line PSN1 was purchased from American Type Culture Collection (Rockville, MD, USA). SUIT2 was supplied by Dr. Oku (Shizuoka University, Shizuoka, Japan). Both of these cell lines were maintained in a DMEM (SIGMA, St. Louis, MO, USA) supplemented with 10% fetal calf serum (Tissue Culture Biologicals, CA, USA), penicillin, streptomycin and amphotericin B (SIGMA) in a 5% CO₂ atmosphere at 37°C.

### Linker-SN-38 complex and immune conjugate

An antibody-prodrug complex was prepared in the same manner as Example 1, and its concentration was determined using the Bradford method (Bio-Rad Protein Assay, 500-0006JA, Bio-Rad Laboratories, Inc.). The number of thiol residues was quantified by DNTP. The ratio of each drug (SN-38)/antibody was determined by comparing the free thiol and thiol residues (ranged from 6.7 to 8.4).

### Immunohistochemistry

Resected tissue was fixed in 1% para-formaldehyde solution at 4°C for 5 hours. The tissue was washed with PBS and blocked with a blocking solution (PBS containing 0.1% bovine serum albumin and 0.1% Triton-X 100) at room temperature for 1 hour. Human samples were purchased from US Biomax, Inc. (Rockville, MD, USA) and BioChain Institute, Inc. (Hayward, CA, USA). The tissue, along with an anti-EpCAM antibody (B8-4) and an anti-collagen IV antibody (35-4 or LSL-LB-1403) as the primary antibodies, was incubated at room temperature for 90 minutes. After being washed with PBS three times, the tissue, along with Alexa 488-labeled antimouse IgG (Invitrogen) and Alexa 555-labeled anti-rat IgG (Invitrogen) as the secondary antibodies, was incubated at room temperature for 60 minutes. After being washed with PBS three times, the tissue was incubated with a PBS containing DAPI (Invitrogen). Alexa 555-labeled anti-rat IgG (Invitrogen) or antihuman IgG (Invitrogen) was used to detect the antibody prodrug SN-38 injected into the tumors. The tissue was covered with a cover slip in a mounting solution (Vector Laboratory). Fluorescent images were obtained using the digital high-definition microscopic SYSTEM BZ-9000 (Keyence Corporation, Osaka, Japan) or the laser scanning microscope system LSM 710 (Carl Zeiss).

### Animal model and antitumor action

Female BALB/c nude mice (5-week-old) were purchased from SLC Japan (Shizuoka, Japan). 2×10⁶ cells were subcutaneously inoculated to each mouse in their flank. Tumor size (length (L) and width (W)) was measured every four days, and tumor volume was calculated using (LxW²)/2. All animal treatments were conducted in compliance with the guidelines for the management and use of laboratory animals established by the Animal Experimentation Committee of the National Cancer Center. These guidelines conform to the legally mandatory ethical standards, meeting the guidelines for the use of laboratory animals in Japan. When the mean tumor volume became about 90 mm³ (PSN1) or about 70 mm³ (SUIT2), the mice were randomly allocated to four groups each of which consisted of five animals. The immune conjugate was administered on the day of tail vein injection. An injection dose of the antibody-SN-38 prodrug equal to the dose of SN-38 was determined by a calculation based on the ratio of each drug (SN-38)/antibody.

### (Results)

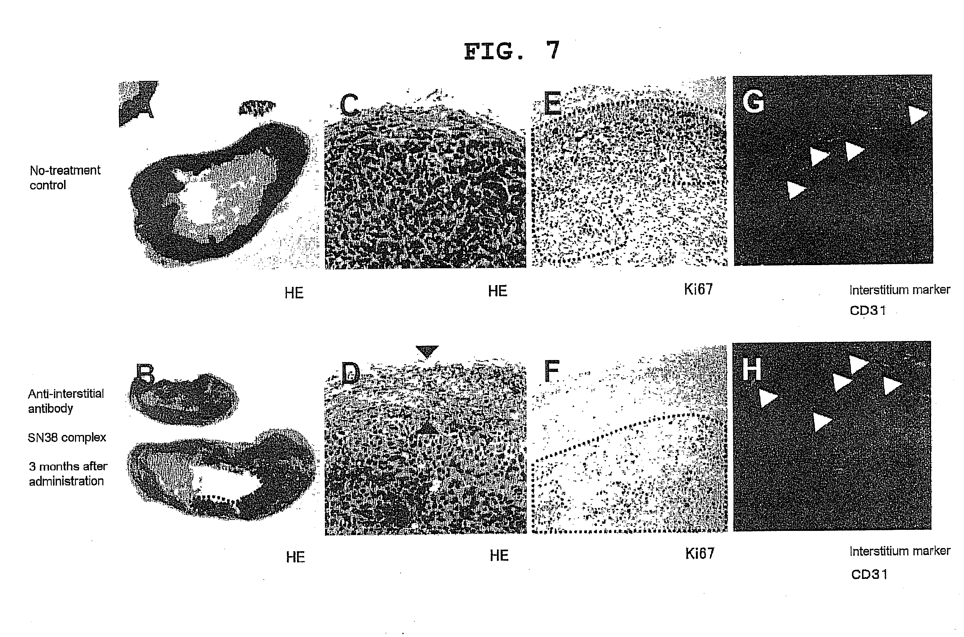

Results are shown in Fig. 7. In Fig. 7, A, C, E and G show tumors not receiving the complex, and B, D, F and H show tumors as of 3 months after administration of the complex. A to D are SUIT2 tumors stained with hematoxylin and eosin; in B, the portion encircled by the dotted line included surviving cells. In D, the portion between the tips of the black triangles indicates the width of the fibrous coat formed. Tumor growth was examined using immunochemical staining with Ki67; results are shown in E and F. In G and H, the tumor blood vessels and their marks were examined by double staining for CD31 and collagen IV. Major positive portions are encircled with dotted lines.
As is often seen in murine models of heterologous transplantation, both of the tumors produced central necrosis due to a reduction in blood flow; however, the control tumors grew, whereas the tumors receiving the complex did not grow (see A and B). Observed only in the tumors receiving the complex were a large necrotic macula and the formation of dense fibrous coat (see C and D). Although most of the Ki67-positive grown tumors were observed on the control boundaries, only a few were also observed at the centers of the tumors receiving the complex (see E and F). Furthermore, CD31-positive endothelial cells formed blood vessels that feed tumors (tumor feeding vessels) in the boundary regions, but unlike in the control, no such vessels were observed in the tumors receiving the complex. Collagen-positive circular rings were seen as blood vessel marks in the boundary regions of the tumors receiving the complex (see G and H).
The results above suggested that a higher antitumor effect can be achieved in vivo when administering a complex comprising a substance having specific binding affinity for a component of tumor interstitium (for example, antibody collagen IV antibody) and an antitumor compound bound thereto, than when administering an antitumor compound without using a targeting substance. It was also shown that by administering a complex comprising a substance having specific binding affinity for a component of tumor interstitium (for example, antibody collagen IV antibody) and an antitumor compound bound thereto, the formation of blood vessels that feed tumors at the boundary regions of tumors is suppressed.

### (Example 5)

### Synthesis of branched linker

### (Step 1)

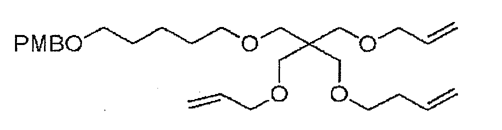

### 1-((5-(3-(allyloxy)-2-(allyloxymethyl)-2-((but-3-enyloxy)methyl)propoxy)pentyloxy)methyl)-4-methoxybenzene

NaH (2.31 g, 160 mmol) was added to a solution of 3-(allyloxy)-2-(allyloxymethyl)-2-((but-3-enyloxy)methyl)propan-1-ol (14.85 g, 31.17 mmol) in DMF (30 mL) at room temperature. One hour later, the bromide compound 1-((5-bromopentyloxy)methyl)-4-methoxybenzene (16.7 g, 58.04 mmol) was added. The flask was twice washed with DMF (3 mL). The mixture was stirred at 55°C for 1 hour. After cooling to room temperature, N,N-dimethyl 1,3-propanediamine (10 mL) was added. One hour later, the mixture was diluted with saturated NH₄Cl and EtOAc. The aqueous layer was extracted with EtOAc. The combined layer was washed with saturated saline. The organic layer was dried over Na₂SO₄ and filtered. After evaporation, the residue was purified using a silica gel column (hexane:EtOAc 9:1-4:1) to yield an ether compound of the formula above as a colorless oily substance (8.60 g, 58%). ¹H-NMR d 7.23 (dd, J = 6.4 Hz, 2.4 Hz, 2H), 6.5 (dd, J = 6.4 Hz, 2.4 Hz, 2H), 5.85 (m, 3H), 5.23 (d, J = 17.2 Hz, 3H), 5.11 (d, J = 10.4 Hz, 3H), 4.41 (s, 2H), 3.93 (m, 6H), 3.78 (s, 3H), 3.42 (s, 8H) 3.39-3.36 (m, 4H), 1.60-1.51 (m, 4H), 1.38 (m, 2H), ¹³C-NMR d 135.21, 129.11, 115.97, 113.69, 72.53, 72.26, 71.33, 7.13, 69.60, 69.42, 55.30, 45.44, 29.65, 29.50, 22.94.

### (Step 2)

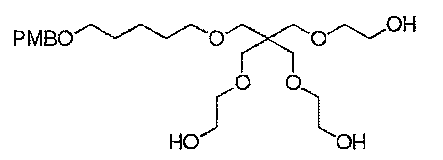

### 2,2'-(2-((2-hydroxyethoxy)methyl)-2-((5-(4-methoxybenzyloxy)pentyloxy)methyl)propane-1,3-diyl)bis(oxy)diethanol

A solution of the triallyl compound 1-((5-(3-(allyloxy)-2-(allyloxymethyl)-2-((but-3-enyloxy)methyl)propoxy)pentyloxy)methyl)-4-methoxybenzene (8.60 g, 18.06 mmol) obtained in the step 1 in CH₂Cl₂ (200 mL) and MeOH (200 mL) was aerated with ozone at -78°C until the reaction mixture turned light blue. After the ozone was replaced with gaseous oxygen, NaBH₄ (8.60 g, 210 mmol) was added in several divided portions. The reaction mixture was gradually warmed, after which it was stirred at room temperature overnight. After the reaction liquid was concentrated, the mixture was subjected to liquid-liquid separation between CHCl₃ and saturated NH₄Cl. The aqueous layer was extracted with CHCl₃. The combined layer was washed with saturated saline, dried over Na₂SO₄, and filtered. The solvent was removed under reduced pressure, and the residue was purified by silica gel column chromatography (CHCl₃:MeOH 9:1) to yield a triol compound of the formula above (8.56 g quant.).
¹H-NMR d 7.23 (d, J = 8.8 Hz, 2H), 6.85 (d, J = 8.8 Hz, 2H), 4.41 (s, 2H), 3.84 (s, 3H), 3.79 (s like, 6H), 3.53 (m, 6H), 3.48 (s like, 8H), 3.43-3.37 (m, 4H), 2.86 (s, 3H), 1.58-1.53 (m, 4H), 1.39 (m, 2H); ¹³C-NMR d 129.16, 113.69, 72.56, 72.50, 71.67, 70.46, 70.05, 70.00, 61.4, 55.30, 45.40, 29.56, 29.32, 22.92.

### (Step 3)

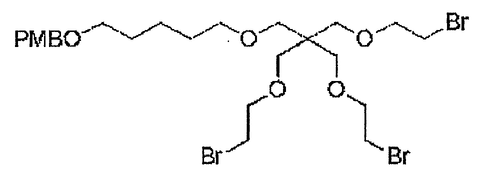

### 1-((5-(3-(2-bromoethoxy)-2,2-bis((2-bromoethoxy)methyl)propoxy)pentyloxy)methyl)-4-methoxybenzene

CBr₄ (8.14 g, 24.51 mmol) was added to a solution of the triol compound (2,2'-(2-((2-hydroxyethoxy)methyl)-2-((5-(4-methoxybenzyloxy)pentyloxy)methyl)propane-1,3-diyl)bis(oxy)diethanol (2.91 g, 6.14 mmol) obtained in the step 2 and PPh₃ (6.43 g, 24.51 mmol) in THF (50 mL) at 0°C in several divided portions. The mixture was stirred at room temperature overnight. Diethyl ether was added to the mixture, and the precipitate was filtered. The filtrate was concentrated and purified by silica gel column chromatography (hexane:EtOAc 9:1-4:1) to yield a tribromide of the formula above (3.4 g, 86%).
¹H-NMR d 7.25 (d, J = 8.4 Hz, 2H), 6.87 (d, J = 8.4 Hz, 2H), 4.42 (s, 2H), 3.80 (s, 3H), 3.74-3.71 (m, 6H), 3.47 (s, 8H), 3.47-3.38 (m, 6H), 1.7-1.50 (m, 4H), 1.40 (m, 2H); ¹³C-NMR d 129.12, 113.66, 72.53, 71.33, 71.14, 70.08, 69.38, 68. 88, 55.29, 45.72, 30.82, 29.63, 22.97.

### (Step 4)

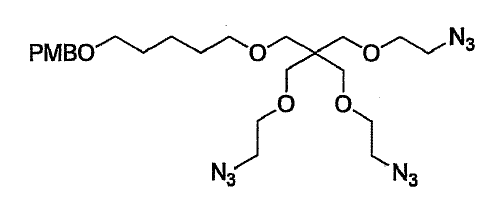

### 1-((5-(3-(2-azidoethoxy)-2,2-bis((2-azidoethoxy)methyl)propoxy)pentyloxy)methyl)-4-methoxybenzene

NaN₃ (5.27 g, 81.08 mmol) was added to a solution of the tribromide (1-((5-(3-(2-bromoethoxy)-2,2-bis((2-bromoethoxy)methyl)propoxy)pentyloxy)methyl)-4-methoxybenzene (3.48 g, 5.27 mmol) obtained in the step 3 in DMF (10 mL), and the mixture was stirred at 50°C for 4 hours. The mixture was diluted with EtOAc and saturated NaHCO₃. The aqueous layer was extracted with EtOAc. The combined layer was washed with saturated saline. After the mixture was dried over Na₂SO₄, the solvent was concentrated under reduced pressure. The residue was purified by silica gel column chromatography (hexane:EtOAc 7:3) to yield a triazide compound of the formula above (2.84 g, 98%).
¹H-NMR d 7.23 (d, J = 8.8 Hz, 2H), 6.85 (d, J = 8.8 Hz, 2H), 4.41 (s, 2H), 3.78 (s, 3H), 3.59 (t, J = 4.8 Hz, 6H), 3.47 (s, 8H), 3.44-3.28 (m, 10H), 1.61-1.55 (m, 4H), 1.39 (m, 2H) ; ¹³C-NMR d 129.04, 113.63, 72.53, 71.32, 70.46, 70.13, 69.71, 68.85, 55.32, 50.86, 45.12, 29.70, 23.04.

### (Step 5)

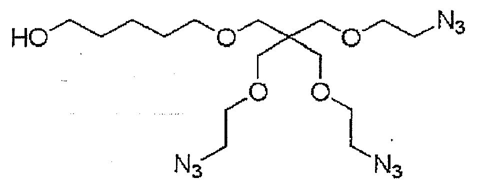

### 5-(3-(2-azidoethoxy)-2,2-bis((2-azidoethoxy)methyl)propoxy)pentan-1-ol

DDQ (1.30 g, 5.75 mmol) was added to a solution of the PMB ether (2.63 g, 4.7 mmol) obtained in the step 4 in CH₂Cl₂ (30 mL) and H₂O (20 mL) at 0°C. After the addition, the ice bath was removed, and the mixture was stirred at room temperature. Five hours later, the reaction was stopped with citrate-buffered solution, and the aqueous layer was extracted with EtOAc. The combined layer was washed with saturated NaHCO₃ and saturated saline. The organic layer was dried over Na₂SO₄. After filtration, the solvent was removed. The residue was purified by silica gel column chromatography (hexane:EtOAc 1:1) to yield an alcohol compound of the formula above (1.55 g, 75%).
¹H-NMR d 3.64-3.58 (m, 8H), 3.46 (s, 6H), 3.41-3.38 (m, 4H), 3.31-3.28 (m, 6H), 1.57-1.55 (m, 4H), 1.41 (m, 2H); ¹³C-NMR d 71.20, 70.46, 69.67, 68.84, 62.92, 50.86, 45.08, 32.57, 29.36, 22.56.

### (Step 6)

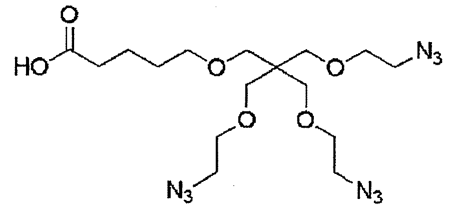

### 5-(3-(2-azidoethoxy)-2,2-bis((2-azidoethoxy)methyl)propoxy)pentanoic acid

The Jones reagent was added to a solution of the alcohol compound (1.55 g, 3. 61 mmol) obtained in the step 5 in acetone (20 mL) at 0°C. The excess portion of the Jones reagent was destroyed with iPrOH, and the precipitate was filtered. After concentration, the residue was purified by silica gel column chromatography (CHCl₃:EtOAc 7:3-1:1) to yield an acid of the formula above (1.37 g, 86%).
¹H-NMR d 3.72 (t, J = 6.0 Hz, 3H), 3.60 (t, J = 4.4 Hz, 4H), 3.47 (s, 8H), 3.44-3.40 (m, 4H), 3.32 (t, J = 4.8 Hz, 4H), 2.83 (t, J = 7.6 Hz, 2H), 1,70 (m, 2H), 1.60 (m, 2H); ¹³C-NMR d177.69, 77.21, 70.77, 70.48, 69.68, 68.96, 50.87, 45.11, 33.55, 28.93, 21.81.

### (Step 7)

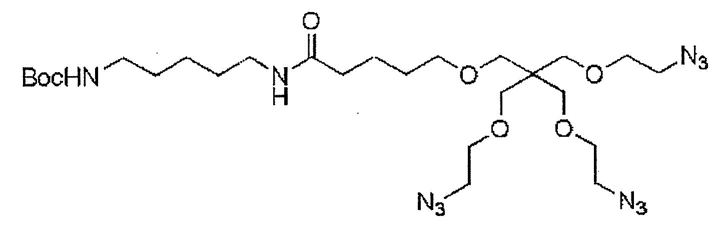

### Tert-butyl 5-(5-(3-(2-azidoethoxy)-2,2-bis((2-azidoethoxy)methyl)propoxy)pentanamido)pentylcarbamate

WSCDI (1.47 g, 7.69 mmol) was added to a solution of the acid obtained in the step 6 (1.65 g, 3.85 mmol), N-(tert-butoxycarbonyl)-1,5-diaminopentane (1.56 g, 7.69 mmol) and HOBt (1.03 g, 7.60 mmol) in CH₂Cl₂ (30 mL) at 0°C. The mixture was stirred at room temperature overnight and diluted with CHCl₃ and saturated NH₄Cl. The aqueous layer was extracted with CHCl₃. The combined layer was washed with saturated NaHCO₃ and saturated saline and dried over Na₂SO₄ to concentrate the solvent. The residue was purified by silica gel column chromatography (CHCl₃: EtOAc 1:1 - EtOAc only) to yield an amide compound of the formula above (2.18 g, 90%).
¹H-NMR d 5.60 (bs, 1H), 4.55 (bs, 1H), 3.72 (t, J = 6.0 Hz, 2H), 3.62 (t, J = 4.8 Hz, 4H), 3.47-3.41 (m, 12H), 3.30 (t, J = 4.8 Hz, 4H), 3.22 (q, J = 6.4 H, 2H), 3.10 (m, 2H), 2.17 (t, J = 7.2 Hz, 2H), 1.70-1.46 (m, 7H), 1.43 (s, 9H), 1.34 (m, 3H), ¹³C-NMR d172.64, 155.87, 77.21, 71.15, 71.13, 70.99, 70.94, 70.50, 69.73, 69.69, 69.36, 68.97, 50.91, 45.35, 45.11, 40.35, 39.36, 36.56, 31.00, 29.88, 20.40, 29.23, 28.55, 24.09, 22.77.

### (Step 8)

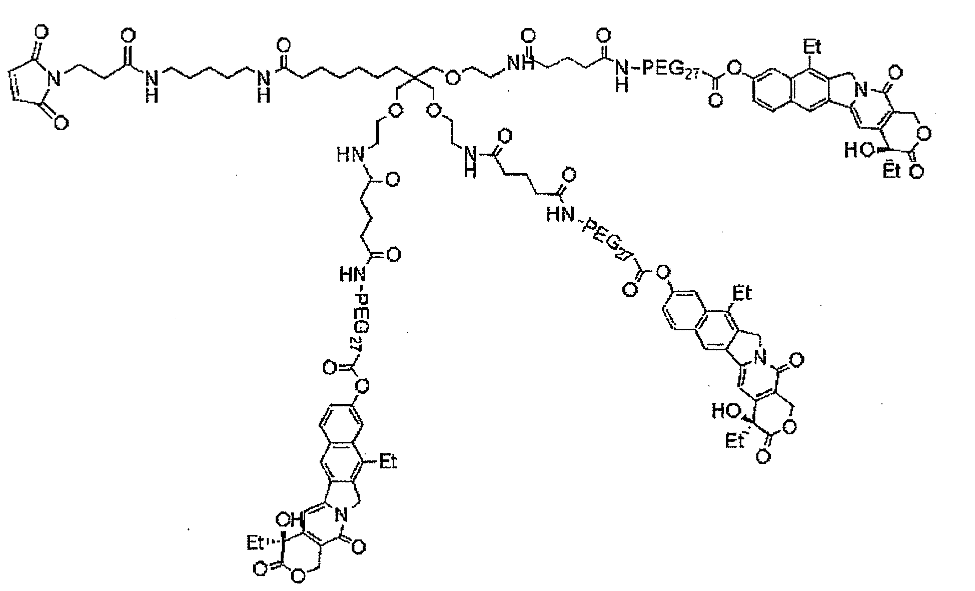

The Boc compound of SN38-PEG obtained in Example 1-1 (0.52 g, 0. 270 mmol) was dissolved in CH₂Cl₂ (20 mL); TFA (2 mL) was added, and the mixture was stirred at room temperature for 2 hours. The reaction liquid was concentrated under reduced pressure; toluene was added, and the liquid was further concentrated, and dried in a vacuum. This residue was dissolved in CH₂Cl₂ (20 mL); iPr₂NEt (0.57 mmol, 3.24 mmol) was added at 0°C, anhydrous succinimide (30 mg, 0.297 mmol) was added, and the mixture was stirred at room temperature overnight. The reaction liquid was purified by LH20 (CHCl₃:MeOH 1:1) and silica gel column chromatography (CHCl₃:MeOH 9:1-4:1).
Meanwhile, triphenyl phosphine (105 mg, 0.40 mmol) was added to a solution of the triazide (63 mg, 0.10 mmol) synthesized in the step 7 in dioxane (1 mL) and water (1 mL), and the mixture was stirred in a nitrogen atmosphere at room temperature overnight.
The reaction liquid was concentrated; the above carboxylic acid was dissolved in CH₂Cl₂ (10 mL) and added, and HOBt (54 mg, 0.4 mmol) and WSCDI (76 mg, 0.40 mmol) were added thereto. The reaction liquid was stirred at room temperature overnight, and the reaction liquid was purified by LH20 (CHCl₃:MeOH 1:1) and silica gel column chromatography (CHCl₃:MeOH 9:1-4:1) to yield 0.22 g. This was dissolved in CH₂Cl₂ (4.5 mL) and TFA (0.5 mL); the reaction liquid was stirred at room temperature for 1 hour, after which it was concentrated under reduced pressure. This was dissolved in CH₂Cl₂ (1 mL); iPr₂NEt (0.1 mL) were added, and N-succimidyl 3-maleimidopropionate (13 mg, 0.470 mmol) was added. The reaction liquid was stirred at room temperature for 2 hours, and this was purified by LH20 (CHCl₃:MeOH 1:1) and silica gel column chromatography (CHCl₃:MeOH 9:1-4:1) to yield 65 mg of the desired product.

### (Reference Example 3)

The VL and VH portions of antibodies were cloned from hybridoma clones that produce an anti-collagen type IV antibodies, 35-4 (rat antimouse collagen type IV antibody IgG2a), 6-1 (mouse antihuman collagen type IV antibody IgM), 6-2 (mouse antihuman collagen type IV antibody IgM) and 56P-1 (mouse antihuman collagen type IV antibody IgM), and the nucleotide sequences were determined.

With reference to the method of Gilliland et al. (Tissue Antigen 1996: 47: 1-20), primers were designed on the basis of particular sequences in the constant regions, and the VL and VH were acquired by the 5'-RACE method and cloned into Promega's pGEM-T Easy vector (TA cloning). Since a specific single band was detected in all these clones, each was cloned into the pGEM-T Easy vector by the TA cloning method, and analyzed for base sequences.

### (Results)

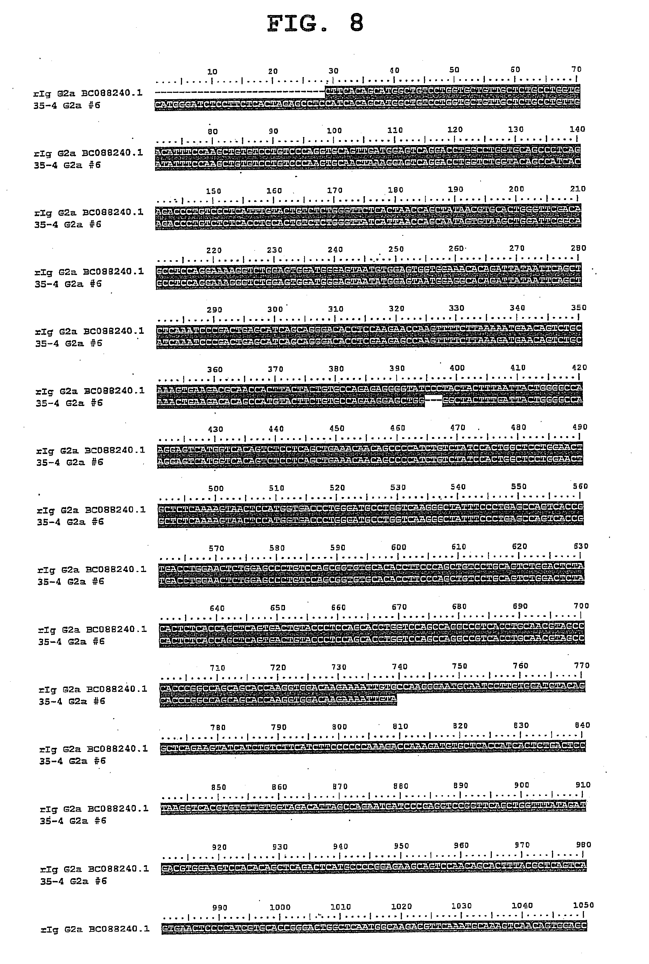

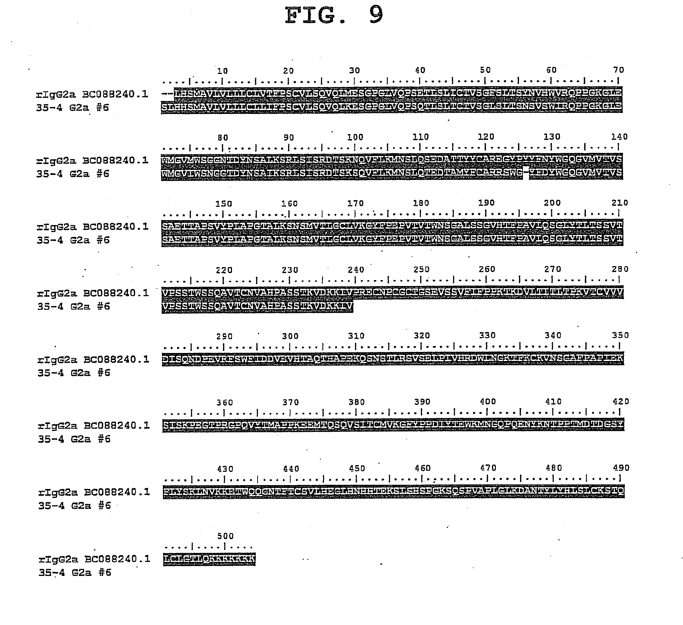

The base sequence of the VH portion of the heavy chain G2a obtained from the hybridoma clone 35-4 is shown by SEQ ID NO:1. Results of alignment of the rIgG2a clone BC088240.1 to the corresponding cDNA sequence (SEQ ID NO:9) are shown in Fig. 8.
The amino acid sequence deduced from the base sequence of the VH portion of the heavy chain G2a obtained from the hybridoma clone 35-4 is shown by SEQ ID NO:12. Results of alignment of the IgG2a clone BC088240.1 to the corresponding amino acid sequence (SEQ ID NO:20) are shown in Fig. 9.

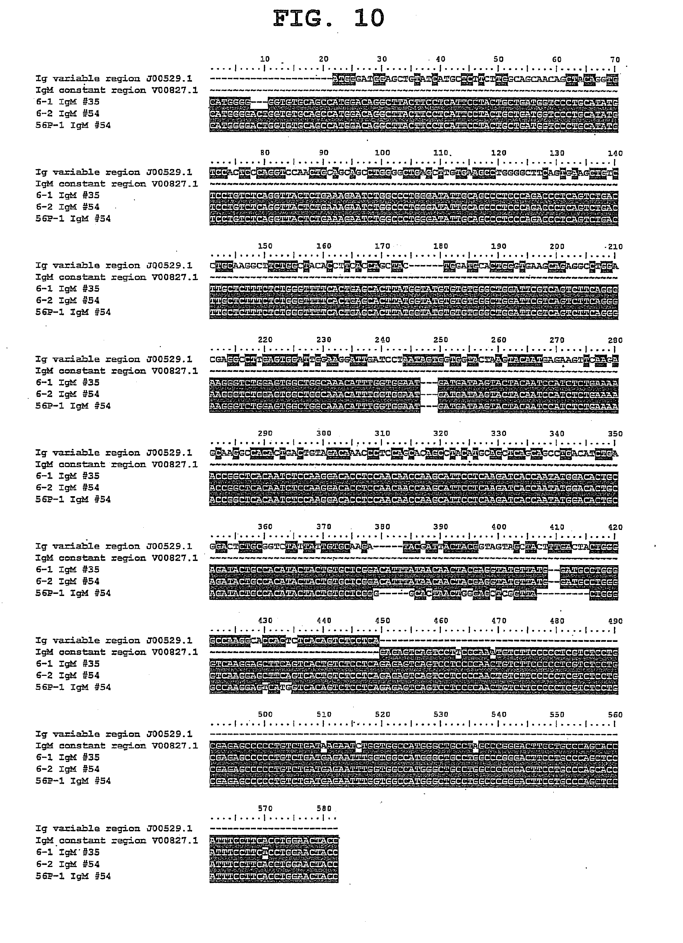

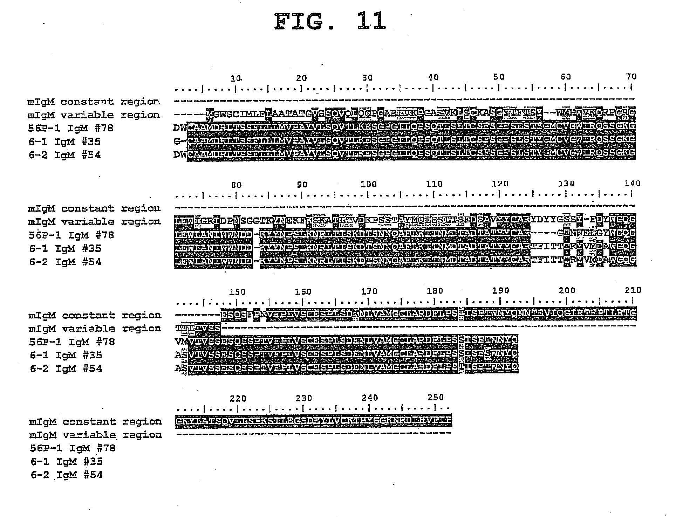

The base sequences of the VH portions of the heavy chains Mu obtained from the hybridoma clones 6-1, 6-2 and 56P-1 are shown by SEQ ID NO:3, 5 and 7, respectively. Results of alignment of the IgM variable region clone J00529.1 and the IgM constant region clone V00827.1 to the corresponding cDNA sequence (SEQ ID NO:10) are shown in Fig. 10.
The amino acid sequences deduced from the base sequences of the VH portions of the heavy chains Mu obtained from the hybridoma clones 6-1, 6-2 and 56P-1 are shown by SEQ ID NO:14, 16 and 18, respectively. Results of alignment of the IgM variable region clone J00529.1 and the IgM constant region clone V00827.1 to the corresponding amino acid sequence (SEQ ID NO:21) are shown in Fig. 11.

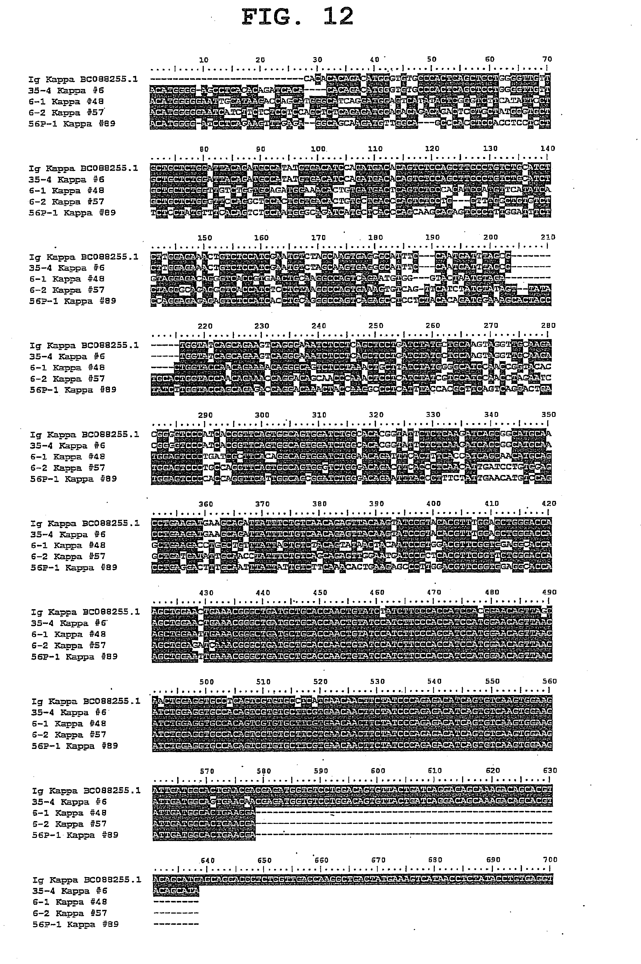

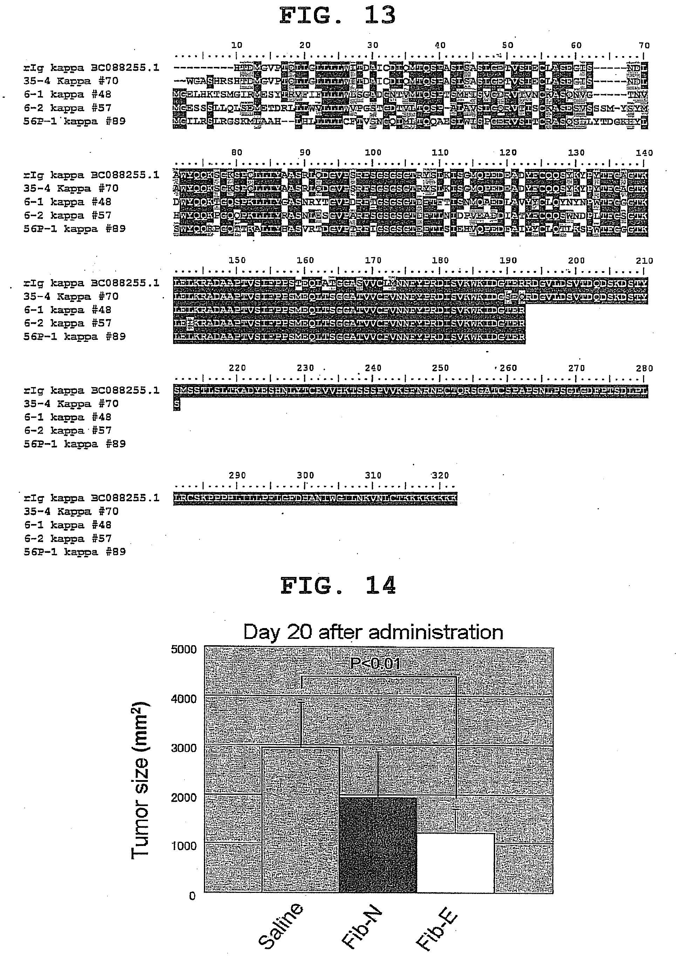

The base sequences of the VL portions of the κ chains obtained from the hybridoma clones 35-4, 6-1, 6-2 and 56P-1 are shown by SEQ ID NO:2, 4, 6 and 8, respectively. Results of alignment of the κ chain clone BC088255.1 to the corresponding cDNA sequence (SEQ ID NO:11) are shown in Fig. 12.
The amino acid sequences deduced from the base sequences of the VL portions of the κ chains obtained from the hybridoma clones 35-4, 6-1, 6-2 and 56P-1 are shown by SEQ ID NO:13, 15, 17 and 19, respectively. Results of alignment of the κ chain clone BC088255.1 to the corresponding amino acid sequence (SEQ ID NO:22) are shown in Fig. 13.

The lengths of VL and VH are shown to be normally about 110 amino acids; it is thought that the full-length cDNA sequences of the VL and VH were obtained because the sequences cloned by the 5'-RACE method in this experiment were similar in length. By using these sequences obtained, it is possible to design and prepare chimeric antibodies and humanized antibodies by conventional methods.

### (Reference Example 4)

Surgical specimens of human pancreatic cancer were stained with an antihuman fibrin antibody (generated by the National Cancer Center). Many fibrin masses were noted in the interstitium in tumor tissue.

Meanwhile, the distribution of interstitial fibrin in tumors that had developed from the human colorectal cancer line HT29 that had been subcutaneously transplanted to nude mice was examined by staining with an antimouse fibrin antibody (generated by the National Cancer Center). Abundant fibrin was noted in the interstitium in tumor tissue.

### (Reference Example 5)

### In vivo imaging of antibody

The accumulation of an antimouse fibrin antibody (generated by the National Cancer Center) in tumors was investigated.
HT29 was transplanted to the back of a BALB/c nude mouse; 10 days after the transplantation, the antimouse fibrin antibody (labeled with IRDye800 (Li-Cor)) was administered via a tail vein of the mouse. The distribution of the antibody after the administration was analyzed using the in vivo imaging apparatus OV110 (Olympus).
Even after the administration, accumulation of the antimouse fibrin antibody in tumor tissue was noted, demonstrating that the antifibrin antibody is highly cumulative in tumors.

The results above suggested that using a substance having specific binding affinity for fibrin for targeting makes it possible to allow an antitumor compound to stay in tumor tissue for a long period.

### (Example 6)

### Production of antifibrin antibody-SN-38 complex (a mode of ester bond)

By converting human fibrinogen to fibrin, and immunizing a mouse with the human fibrin, a monoclonal antibody that specifically recognizes human fibrin was obtained. This antibody also exhibited a cross-reaction with mouse fibrin. The cDNA sequence of the variable portion of this antibody was clarified, and a chimeric antibody comprising the variable region and the human Fc portion was generated.
In the same manner as Example 1, an antifibrin antibody-SN-38 complex was produced. In the complex obtained here, SN-38 is bound to the linker via an ester bond. A schematic diagram of the complex obtained is shown below.

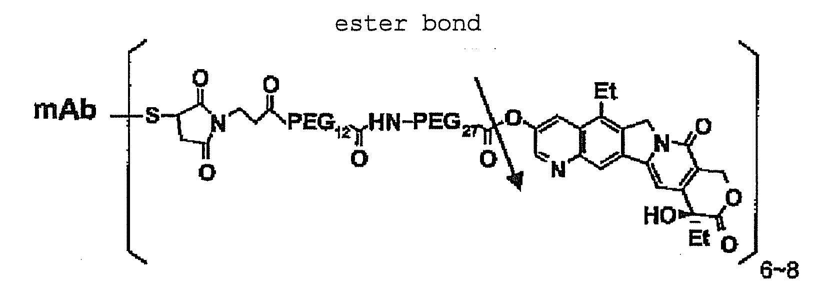

### (Example 7)

### Production of antifibrin antibody-SN-38 complex (a mode of carbamate bond)

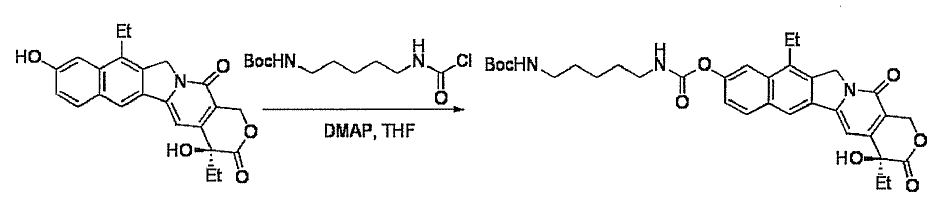

Triphosgene (483 mg, 1.63 mmol) was added to a solution of tert-butoxycarbonyl amino pentanamine (1.00 g, 4.95 mmol) and triethylamine (1.3 mL) in methylene chloride (10 mL) at 0°C in several divided portions. After the reaction liquid was stirred for 2 hours, the precipitate was filtered through Celite in a nitrogen atmosphere. The filtrate was added to a suspension of SN-38 (1.21 g, 3.10 mmol) and DMAP (375 mg, 3.10 mmol) in THF (20 mL) at 0°C. The reaction liquid was stirred at room temperature in a nitrogen atmosphere for one day. The reaction liquid was purified by molecular sieve column (LH20 CHCl₃:MeOH 1:1) and silica gel column chromatography (CHCl₃:MeOH 20:1-10:1) to yield a carbamate (828 mg, 43%) as a colorless foamy substance.

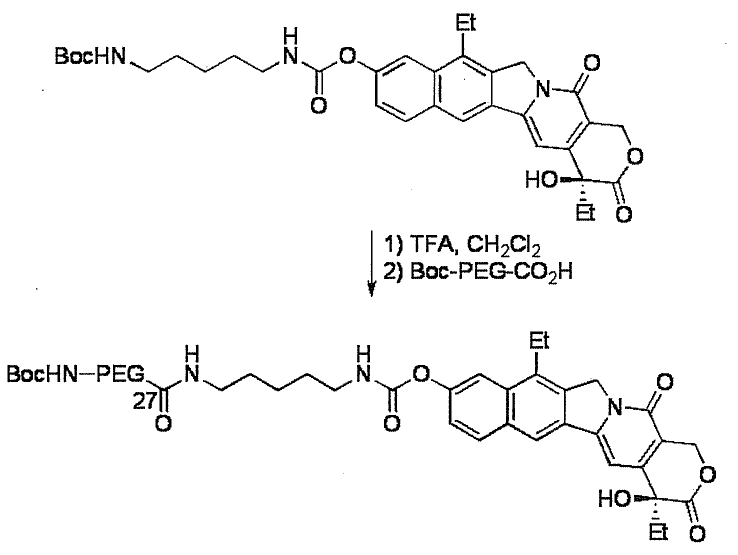

The Boc compound (52.0 mg, 0.084 mmol) was dissolved in CH₂Cl₂-TFA (1:10, 1.1 mL) and stirred at room temperature for 3 hours. The toluene (50 mL) reaction liquid was concentrated. Methylene chloride (2 mL) was added to the residue, and i-Pr₂NEt (0.3 mL) and PEG-succimide (200 mg) were added at 0°C. After stirring at room temperature for 3 hours, the reaction liquid was purified by LH20 (MeOH:CHCl₃ 1:1) and silica gel column chromatography (CHCl₃:MeOH 10:1) to yield 111.9 mg (69%) of a PEG addition product.

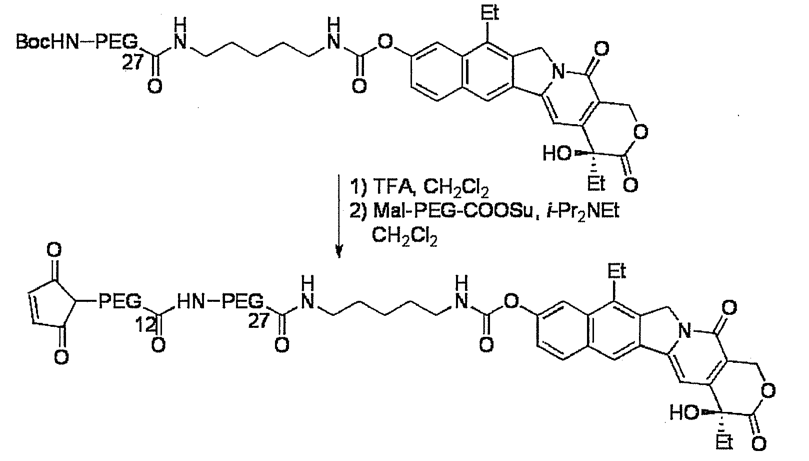

The Boc compound (330 mg, 0.171 mmol) was dissolved in methylene chloride (4 mL), and TFA (0.4 mL) was added; the mixture was stirred for 2 hours. Toluene (50 mL) was added, and the reaction liquid was concentrated. The residue was dissolved in methylene chloride (4 mL), and i-Pr₂NEt (0.15 mL, 0.884 mmol) and MAL-PEG₁₂-succimide (148 mg, 0.171 mmol) were added at 0°C. After stirring for 2 hours, the reaction liquid was purified by SX-4 (toluene) and silica gel column chromatography (CHCl₃:MeOH 20:1-10:1) to yield 157 mg of the desired product.
In the complex obtained here, SN-38 is bound to the linker via a carbamate bond. A schematic diagram of the complex obtained is shown below.

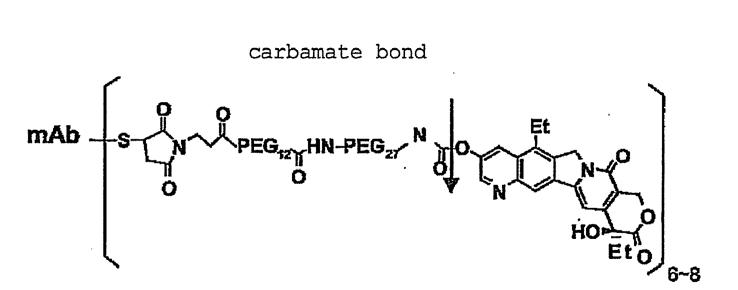

### (Example 8)

### In vivo antitumor effect

The Human colorectal cancer HT29 was subcutaneously transplanted to nude mice; when the tumor diameter reached 6 mm, the antifibrin antibody-SN-38 complex obtained in Example 6 (hereinafter Fib-E), the antifibrin antibody-SN-38 complex obtained in Example 7 (hereinafter Fib-N), or physiological saline (control) was intravenously administered (3 mg/kg each, based on SN-38), and the size of the tumor diameter on day 20 after the administration was monitored. Results are shown in Fig. 14.
Although all antifibrin antibody-SN-38 complexes exhibited a high antitumor effect, Fib-E exhibited a higher antitumor effect than Fib-N.

The results above suggested that even when a substance having specific binding affinity for fibrin was used for targeting, a high antitumor effect could be achieved. It was also suggested that by binding an antitumor compound to the complex via an ester bond, a higher antitumor effect would be expectable.

### Industrial Applicability

According to the present invention, a complex is provided that stays specifically in tumor interstitium for a long time to exhibit an excellent antitumor effect. In particular, using the complex of the present invention makes it possible to allow an antitumor compound having a time-dependent antitumor effect to effectively exhibit the antitumor effect. Also, because the complex of the present invention acts on tumor blood vessels and/or tumor cells, a remarkably higher antitumor effect is expectable than conventional complexes.

This application is based on patent application Nos. 2008-293930 filed in Japan (filing date: November 17, 2008) and 2009-125871 filed in Japan (filing date: May 25, 2009), the contents of which are incorporated in full herein.

## Claims

1. A complex comprising a substance having specific binding affinity for stroma and an antitumor site bound to the substance.

2. The complex according to claim 1, wherein the substance having specific binding affinity for stroma and the antitumor site are bound via a linker.

3. The complex according to claim 1, wherein the antitumor site is an antitumor compound.

4. The complex according to claim 1, wherein the antitumor site is a functional structure capable of sustained release of an antitumor compound.

5. The complex according to claim 4, wherein the functional structure capable of sustained release of an antitumor compound is a liposome or micelle containing an antitumor compound.

6. The complex according to claim 1, wherein the substance having specific binding affinity for stroma is an antibody having specific binding affinity for stroma or a binding fragment thereof.

7. The complex according to claim 1, wherein the stroma is a collagen.

8. The complex according to claim 7, wherein the collagen is type IV collagen.

9. The complex according to claim 1, wherein the stroma is fibrin.

10. The complex according to claim 2, wherein the linker and the antitumor site are bound via an ester bond or a carbamate bond.

11. The complex according to claim 2, wherein the linker and the antitumor site are bound via a carbonate bond or a thiocarbamate bond.

12. A tumor therapeutic agent comprising the complex according to claim 1.

13. An inhibitor of tumor blood vessel formation comprising the complex according to claim 1.

14. A method of treating a tumor in a mammal, comprising administering an effective amount of the complex according to claim 1 to the mammal.

15. A method of inhibiting the formation of tumor blood vessels in a mammal, comprising administering an effective amount of the complex according to claim 1 to the mammal.

16. The complex according to claim 1, wherein the complex is to be used in the treatment of a tumor.

17. The complex according to claim 1, wherein the complex is to be used in the inhibition of the formation of tumor blood vessels.


SEQUENCE LISTING

<110>  JAPAN HEALTH SCIENCES FOUNDATION
       RIKEN

<120>  Novel cancer targetting therapy using complex of substance having
        specific binding ability to stroma consisting factor and
       antitumor compound

<130>  091476

<150>  JP 2008-293930
<151>  2008-11-17

<150>  JP 2009-125871
<151>  2009-05-25

<160>  24

<170>  PatentIn version 3.3

<210>  1
<211>  736
<212>  DNA
<213>  Rattus norvegicus

<400>  1
catgggatct ccttctcact agagcctcca tcacagcatg gctgtcctgg tgctgttgct      60

ctgcctgttg atatttccaa gctgtgtcct gtcccaagtg caactaaagg agtcaggacc     120

tggtctggta cagccatcac agaccctgtc tctcacctgc actgtctctg ggttatcatt     180

aaccagcaat agtgtaagct ggattcggca gcctccagga aagggtctgg agtggatggg     240

agtaatatgg agtaatggag gcacagatta taattcagct atcaaatccc gactgagcat     300

cagcagggac acctcgaaga gccaagtttt cttaaagatg aacagtctgc aaactgaaga     360

cacagccatg tacttctgtg ccagaaggag ctggggctac tttgattact ggggccaagg     420

agtcatggtc acagtctcct cagctgaaac aacagcccca tctgtctatc cactggctcc     480

tggaactgct ctcaaaagta actccatggt gaccctggga tgcctggtca agggctattt     540

ccctgagcca gtcaccgtga cctggaactc tggagccctg tccagcggtg tgcacacctt     600

cccagctgtc ctgcagtctg gactctacac tctcaccagc tcagtgactg taccctccag     660

cacctggtcc agccaggccg tcacctgcaa cgtagcccac ccggccagca gcaccaaggt     720

ggacaagaaa attgta                                                     736


<210>  2
<211>  618
<212>  DNA
<213>  Rattus norvegicus

<400>  2

acatggggag cctcacacag atcacacaca gacatgggtg tgcccactca gctcctgggg 60

ttgttgctgc tctggattac agatgccata tgtgacatcc agatgacaca gtctccagct 120

tccctgtctg catctcttgg agaaactgtc tccatcgaat gtctagcaag tgagggcatt 180

tccaatgatt tagcgtggta tcagcagaag tcagggaaat ctcctcagct cctgatctat 240

gctgcaagta ggttgcaaga cggggtccca tcacggttca gtggcagtgg atctggcaca 300

cggtattctc tcaagatcag cggcatgcaa cctgaagatg aagcagatta tttctgtcaa 360

cagagttaca agtatccgta cacgtttgga gctgggacca agctggaact gaaacgggct 420

gatgctgcac caactgtatc catcttccca ccatccatgg aacagttaac atctggaggt 480

gccacagtcg tgtgcttcgt gaacaacttc tatcccagag acatcagtgt caagtggaag 540

attgatggca gtgaacaacg agatggtgtc ctggacagtg ttactgatca ggacagcaaa 600

gacagcacgt acagcata 618

<210> 3
<211> 574
<212> DNA
<213> Mus musculus

<400> 3
catggggggt gtgcagccat ggacaggctt acttcctcat tcctactgct gatggtccct 60

gcatatgtcc tgtctcaggt tactctgaaa gaatctggcc ctgggatatt gcagccctcc 120

cagaccctca gtctgacttg ctctttctct gggttttcac tgagcactta tggtatgtgt 180

gtgggctgga ttcgtcagtc ttcagggaag ggtctggagt ggctggcaaa catttggtgg 240

aatgatgata agtactacaa tccatctctg aaaaaccggc tcacaatctc caaggacacc 300

tccaacaacc aagcattcct caagatcacc aatatggaca ctgcagatac tgccacatac 360

tactgtgctc ggacatttat aacaactacg aggtatgtta tggatgcctg gggtcaagga 420

gcttcagtca ctgtctcctc agagagtcag tcctccccaa ctgtcttccc cctcgtctcc 480

tgcgagagcc ccctgtctga tgagaatttg gtggccatgg gctgcctggc ccgggacttc 540

ctgcccagct ccatttcctt ctcctggaac tacc 574

<210> 4
<211> 563
<212> DNA
<213> Mus musculus

<400> 4
acatggggga attgcataag accagcatgg gcatcaggat ggagtcatat actcgggtct 60

tcatattcct gctgctctgg ttgtctggtg cagatggaaa cactgtgatg actcagtctc 120

ccacatccat gttcatatca gtaggagaca gggtcaccgt gaactgcaag gccagtcaga 180

atgtgggtac taatgtagac tggtaccaac agaaaacagg gcagtctcct aaactgctta    240

tctatggggc atccaaccgg tacactggag tccctgatcg cttcacaggc agtggatctg    300

gaacagattt cactttcacc atcagcaaca tgcaggctga agacctggct gtttattact    360

gtctacagta taactacaat ccgtggacgt tcggtggagg caccaagctg gaattgaaac    420

gggctgatgc tgcaccaact gtatccatct tcccaccatc catggaacag ttaacatctg    480

gaggtgccac agtcgtgtgc ttcgtgaaca acttctatcc cagagacatc agtgtcaagt    540

ggaagattga tggcactgaa cga                                            563

<210>  5
<211>  577
<212>  DNA
<213>  Mus musculus

<400>  5
catggggact ggtgtgcagc catggacagg cttacttcct cattcctact gctgatggtc     60

cctgcatatg tcctgtctca ggttactctg aaagaatctg gccctgggat attgcagccc    120

tcccagaccc tcagtctgac ttgctctttc tctgggtttt cactgagcac ttatggtatg    180

tgtgtgggct ggattcgtca gtcttcaggg aagggtctgg agtggctggc aaacatttgg    240

tggaatgatg ataagtacta caatccatct ctgaaaaacc ggctcacaat ctccaaggac    300

acctccaaca accaagcatt cctcaagatc accaatatgg acactgcaga tactgccaca    360

tactactgtg ctcggacatt tataacaact acgaggtatg ttatggatgc ctggggtcaa    420

ggagcttcag tcactgtctc ctcagagagt cagtcctccc caactgtctt ccccctcgtc    480

tcctgcgaga gccccctgtc tgatgagaat ttggtggcca tgggctgcct ggcccgggac    540

ttcctgccca gcaccatttc cttcacctgg aactacc                             577

<210>  6
<211>  572
<212>  DNA
<213>  Mus musculus

<400>  6
acatggggga atcatcttct ctcctccagc tctcagagat ggagacagac agactcctgc     60

tatgggtgct gctgctctgg gttccaggct ccactggtga cactgtgctg acccagtctc    120

ctgctttggc tgtgtctcta gggcagaggg tcaccatctc ctgtaaggcc agtgaaagtg    180

tcagttcatc tatgtatagt tatatgcact ggtaccaaca gaaaccagga cagcaaccca    240

aactcctcat ctatcgtgca tccaacctag aatctggagt ccctgccagg ttcagtggca    300

gtgggtctgg gacagacttc accctcaaca ttgatcctgt ggaggctgat gatattgcaa    360

cctatttctg tcagcagagt tggaatgatc cgctcacgtt cggttctggg accaagctgg 420

agatcaaacg ggctgatgct gcaccaactg tatccatctt cccaccatcc atggaacagt 480

taacatctgg aggtgccaca gtcgtgtgct tcgtgaacaa cttctatccc agagacatca 540

gtgtcaagtg gaagattgat ggcactgaac ga 572

<210> 7
<211> 565
<212> DNA
<213> Mus musculus

<400> 7
catggggact ggtgtgcagc catggacagg cttacttcct cattcctact gctgatggtc 60

cctgcatatg tcctgtctca ggttactctg aaagaatctg gccctgggat attgcagccc 120

tcccagaccc tcagtctgac ttgctctttc tctgggtttt cactgagcac ttatggtatg 180

tgtgtgggct ggattcgtca gtcttcaggg aagggtctgg agtggctggc aaacatttgg 240

tggaatgatg ataagtacta caatccatct ctgaaaaacc ggctcacaat ctccaaggac 300

acctccaaca accaagcatt cctcaagatc accaatatgg acactgcaga tactgccaca 360

tactactgtg ctcggggcac taactgggag ctcggttact ggggccaagg agtcatggtc 420

acagtctcct cagagagtca gtcctcccca actgtcttcc ccctcgtctc ctgcgagagc 480

cccctgtctg atgagaattt ggtggccatg ggctgcctgg cccgggactt cctgcccagc 540

tccatttcct tcacctggaa ctacc 565

<210> 8
<211> 572
<212> DNA
<213> Mus musculus

<400> 8
acatggggat cctcagaagt ttgagaggca gcaagatgtt ggcagcccac ctccacctcc 60

tccttctcct atgtttcaca gtctccaatg ggcagatcat gctcacccag caagcagagt 120

ccctttggat ttctccagga gagagagtct ccatcacctg cagggccagt cagagcctcc 180

tctacacaga tggaaagcac tacctatctt ggtaccagca gagaccagga caaactacca 240

aggccctcat ttaccacgct tcagtcagga ctgatggagt ccccaccagg ttcattggca 300

gcggatctgg gacagaattt accctttcta ttgaacatgt ccagcctgag gactttgcaa 360

tttattattg tcttcaaaca ctgaagagcc cttggacgtt cggtggaggc accaagctgg 420

aattgaaacg ggctgatgct gcaccaactg tatccatctt cccaccatcc atggaacagt 480

taacatctgg aggtgccaca gtcgtgtgct tcgtgaacaa cttctatccc agagacatca 540

gtgtcaagtg gaagattgat ggcactgaac ga                                    572

<210>  9
<211>  1022
<212>  DNA
<213>  Rattus norvegicus

<400>  9
cttcacagca tggctgtcct ggtgctgttg ctctgcctgg tgacatttcc aagctgtgtc      60

ctgtcccagg tgcagttgat ggagtcagga cctggcctgg tgcagccctc agagaccctg     120

tccctcattt gtactgtctc tgggttctca ctaaccagct ataacgtgca ctgggttcga     180

cagcctccag gaaaaggtct ggagtggatg ggagtaatgt ggagtggtgg aaacacagat     240

tataattcag ctctcaaatc ccgactgagc atcagcaggg acacctccaa gaaccaagtt     300

ttcttaaaaa tgaacagtct gcaaagtgaa gacgcaacca cttactactg tgccagagag     360

gggtatccct actactttaa ttactggggc caaggagtca tggtcacagt ctcctcagct     420

gaaacaacag ccccatctgt ctatccactg gctcctggaa ctgctctcaa aagtaactcc     480

atggtgaccc tgggatgcct ggtcaagggc tatttccctg agccagtcac cgtgacctgg     540

aactctggag ccctgtccag cggtgtgcac accttcccag ctgtcctgca gtctggactc     600

tacactctca ccagctcagt gactgtaccc tccagcacct ggtccagcca ggccgtcacc     660

tgcaacgtag cccacccggc cagcagcacc aaggtggaca agaaaattgt gccaagggaa     720

tgcaatcctt gtggatgtac aggctcagaa gtatcatctg tcttcatctt ccccccaaag     780

accaaagatg tgctcaccat cactctgact cctaaggtca cgtgtgttgt ggtagacatt     840

agccagaatg atcccgaggt ccggttcagc tggtttatag atgacgtgga agtccacaca     900

gctcagactc atgccccgga gaagcagtcc aacagcactt tacgctcagt cagtgaactc     960

cccatcgtgc accgggactg gctcaatggc aagacgttca aatgcaaagt caacagtgga    1020

gc                                                                   1022

<210>  10
<211>  133
<212>  DNA
<213>  Mus musculus

<400>  10
gagagtcagt ccttcccaaa tgtcttcccc ctcgtctcct gcgagagccc cctgtctgat      60

aagaatctgg tggccatggg ctgcctagcc cgggacttcc tgcccagcac catttccttc     120

acctggaact acc                                                        133

<210>  11

<211>  658
<212>  DNA
<213>  Mus musculus

<400>  11
cacacacaga catgggtgtg cccactcagc tcctggggtt gttgctgctc tggattacag      60

atgccatatg tgacatccag atgacacagt ctccagcttc cctgtctgca tctcttggag     120

aaactgtctc catcgaatgt ctagcaagtg agggcatttc caatgattta gcgtggtatc     180

agcagaagtc agggaaatct cctcagctcc tgatctatgc tgcaagtagg ttgcaagacg     240

gggtcccatc acggttcagt ggcagtggat ctggcacacg gtattctctc aagatcagcg     300

gcatgcaacc tgaagatgaa gcagattatt tctgtcaaca gagttacaag tatccgtaca     360

cgtttggagc tgggaccaag ctggaactga aacgggctga tgctgcacca actgtatcta     420

tcttcccacc atccacggaa cagttagcaa ctggaggtgc ctcagtcgtg tgcctcatga     480

acaacttcta tcccagagac atcagtgtca agtggaagat tgatggcact gaacgacgag     540

atggtgtcct ggacagtgtt actgatcagg acagcaaaga cagcacgtac agcatgagca     600

gcaccctctc gttgaccaag gctgactatg aaagtcataa cctctatacc tgtgaggt       658


<210>  12
<211>  238
<212>  PRT
<213>  Rattus norvegicus

<400>  12

Ser Leu His His Ser Met Ala Val Leu Val Leu Leu Leu Cys Leu Leu
1               5                   10                  15


Ile Phe Pro Ser Cys Val Leu Ser Gln Val Gln Leu Lys Glu Ser Gly
            20                  25                  30


Pro Gly Leu Val Gln Pro Ser Gln Thr Leu Ser Leu Thr Cys Thr Val
        35                  40                  45


Ser Gly Leu Ser Leu Thr Ser Asn Ser Val Ser Trp Ile Arg Gln Pro
    50                  55                  60


Pro Gly Lys Gly Leu Glu Trp Met Gly Val Ile Trp Ser Asn Gly Gly
65                  70                  75                  80


Thr Asp Tyr Asn Ser Ala Ile Lys Ser Arg Leu Ser Ile Ser Arg Asp
                85                  90                  95


Thr Ser Lys Ser Gln Val Phe Leu Lys Met Asn Ser Leu Gln Thr Glu

            100                 105                 110

Asp Thr Ala Met Tyr Phe Cys Ala Arg Arg Ser Trp Gly Tyr Phe Asp
        115                 120                 125

Tyr Trp Gly Gln Gly Val Met Val Thr Val Ser Ser Ala Glu Thr Thr
    130                 135                 140

Ala Pro Ser Val Tyr Pro Leu Ala Pro Gly Thr Ala Leu Lys Ser Asn
145                 150                 155                 160

Ser Met Val Thr Leu Gly Cys Leu Val Lys Gly Tyr Phe Pro Glu Pro
                165                 170                 175

Val Thr Val Thr Trp Asn Ser Gly Ala Leu Ser Ser Gly Val His Thr
            180                 185                 190

Phe Pro Ala Val Leu Gln Ser Gly Leu Tyr Thr Leu Thr Ser Ser Val
        195                 200                 205

Thr Val Pro Ser Ser Thr Trp Ser Ser Gln Ala Val Thr Cys Asn Val
    210                 215                 220

Ala His Pro Ala Ser Ser Thr Lys Val Asp Lys Lys Ile Val
225                 230                 235

<210> 13
<211> 204
<212> PRT
<213> Rattus norvegicus

<400> 13

Trp Gly Ala Ser His Arg Ser His Thr Asp Met Gly Val Pro Thr Gln
1               5                   10                  15

Leu Leu Gly Leu Leu Leu Leu Trp Ile Thr Asp Ala Ile Cys Asp Ile
            20                  25                  30

Gln Met Thr Gln Ser Pro Ala Ser Leu Ser Ala Ser Leu Gly Glu Thr
        35                  40                  45

Val Ser Ile Glu Cys Leu Ala Ser Glu Gly Ile Ser Asn Asp Leu Ala
    50                  55                  60

Trp Tyr Gln Gln Lys Ser Gly Lys Ser Pro Gln Leu Leu Ile Tyr Ala
65                  70                  75                  80

Ala Ser Arg Leu Gln Asp Gly Val Pro Ser Arg Phe Ser Gly Ser Gly
                85                  90                  95

Ser Gly Thr Arg Tyr Ser Leu Lys Ile Ser Gly Met Gln Pro Glu Asp
            100                 105                 110

Glu Ala Asp Tyr Phe Cys Gln Gln Ser Tyr Lys Tyr Pro Tyr Thr Phe
        115                 120                 125

Gly Ala Gly Thr Lys Leu Glu Leu Lys Arg Ala Asp Ala Ala Pro Thr
    130                 135                 140

Val Ser Ile Phe Pro Pro Ser Met Glu Gln Leu Thr Ser Gly Gly Ala
145                 150                 155                 160

Thr Val Val Cys Phe Val Asn Asn Phe Tyr Pro Arg Asp Ile Ser Val
                165                 170                 175

Lys Trp Lys Ile Asp Gly Ser Glu Gln Arg Asp Gly Val Leu Asp Ser
            180                 185                 190

Val Thr Asp Gln Asp Ser Lys Asp Ser Thr Tyr Ser
        195                 200

<210>  14
<211>  190
<212>  PRT
<213>  Mus musculus

<400>  14

Gly Cys Ala Ala Met Asp Arg Leu Thr Ser Ser Phe Leu Leu Leu Met
1               5                   10                  15

Val Pro Ala Tyr Val Leu Ser Gln Val Thr Leu Lys Glu Ser Gly Pro
            20                  25                  30

Gly Ile Leu Gln Pro Ser Gln Thr Leu Ser Leu Thr Cys Ser Phe Ser
        35                  40                  45

Gly Phe Ser Leu Ser Thr Tyr Gly Met Cys Val Gly Trp Ile Arg Gln
    50                  55                  60

Ser Ser Gly Lys Gly Leu Glu Trp Leu Ala Asn Ile Trp Trp Asn Asp
65                  70                  75                  80

Asp Lys Tyr Tyr Asn Pro Ser Leu Lys Asn Arg Leu Thr Ile Ser Lys

85                  90                  95

Asp Thr Ser Asn Asn Gln Ala Phe Leu Lys Ile Thr Asn Met Asp Thr
            100                 105                 110

Ala Asp Thr Ala Thr Tyr Tyr Cys Ala Arg Thr Phe Ile Thr Thr Thr
        115                 120                 125

Arg Tyr Val Met Asp Ala Trp Gly Gln Gly Ala Ser Val Thr Val Ser
    130                 135                 140

Ser Glu Ser Gln Ser Ser Pro Thr Val Phe Pro Leu Val Ser Cys Glu
145                 150                 155                 160

Ser Pro Leu Ser Asp Glu Asn Leu Val Ala Met Gly Cys Leu Ala Arg
                165                 170                 175

Asp Phe Leu Pro Ser Ser Ile Ser Phe Ser Trp Asn Tyr Gln
            180                 185                 190

<210>  15
<211>  187
<212>  PRT
<213>  Mus musculus

<400>  15

Met Gly Glu Leu His Lys Thr Ser Met Gly Ile Arg Met Glu Ser Tyr
1               5                   10                  15

Thr Arg Val Phe Ile Phe Leu Leu Leu Trp Leu Ser Gly Ala Asp Gly
            20                  25                  30

Asn Thr Val Met Thr Gln Ser Pro Thr Ser Met Phe Ile Ser Val Gly
        35                  40                  45

Asp Arg Val Thr Val Asn Cys Lys Ala Ser Gln Asn Val Gly Thr Asn
    50                  55                  60

Val Asp Trp Tyr Gln Gln Lys Thr Gly Gln Ser Pro Lys Leu Leu Ile
65                  70                  75                  80

Tyr Gly Ala Ser Asn Arg Tyr Thr Gly Val Pro Asp Arg Phe Thr Gly
                85                  90                  95

Ser Gly Ser Gly Thr Asp Phe Thr Phe Thr Ile Ser Asn Met Gln Ala
            100                 105                 110

Glu Asp Leu Ala Val Tyr Tyr Cys Leu Gln Tyr Asn Tyr Asn Pro Trp
        115                 120                 125

Thr Phe Gly Gly Gly Thr Lys Leu Glu Leu Lys Arg Ala Asp Ala Ala
    130                 135                 140

Pro Thr Val Ser Ile Phe Pro Pro Ser Met Glu Gln Leu Thr Ser Gly
145                 150                 155                 160

Gly Ala Thr Val Val Cys Phe Val Asn Asn Phe Tyr Pro Arg Asp Ile
                165                 170                 175

Ser Val Lys Trp Lys Ile Asp Gly Thr Glu Arg
            180                 185

<210>  16
<211>  191
<212>  PRT
<213>  Mus musculus

<400>  16

Asp Trp Cys Ala Ala Met Asp Arg Leu Thr Ser Ser Phe Leu Leu Leu
1               5                   10                  15

Met Val Pro Ala Tyr Val Leu Ser Gln Val Thr Leu Lys Glu Ser Gly
            20                  25                  30

Pro Gly Ile Leu Gln Pro Ser Gln Thr Leu Ser Leu Thr Cys Ser Phe
        35                  40                  45

Ser Gly Phe Ser Leu Ser Thr Tyr Gly Met Cys Val Gly Trp Ile Arg
    50                  55                  60

Gln Ser Ser Gly Lys Gly Leu Glu Trp Leu Ala Asn Ile Trp Trp Asn
65                  70                  75                  80

Asp Asp Lys Tyr Tyr Asn Pro Ser Leu Lys Asn Arg Leu Thr Ile Ser
                85                  90                  95

Lys Asp Thr Ser Asn Asn Gln Ala Phe Leu Lys Ile Thr Asn Met Asp
            100                 105                 110

Thr Ala Asp Thr Ala Thr Tyr Tyr Cys Ala Arg Thr Phe Ile Thr Thr
        115                 120                 125

Thr Arg Tyr Val Met Asp Ala Trp Gly Gln Gly Ala Ser Val Thr Val

130                 135                 140

Ser Ser Glu Ser Gln Ser Ser Pro Thr Val Phe Pro Leu Val Ser Cys
145                 150                 155                 160

Glu Ser Pro Leu Ser Asp Glu Asn Leu Val Ala Met Gly Cys Leu Ala
                165                 170                 175

Arg Asp Phe Leu Pro Ser Thr Ile Ser Phe Thr Trp Asn Tyr Gln
            180                 185                 190

<210>  17
<211>  190
<212>  PRT
<213>  Mus musculus

<400>  17

Met Gly Glu Ser Ser Ser Leu Leu Gln Leu Ser Glu Met Glu Thr Asp
1               5                   10                  15

Arg Leu Leu Leu Trp Val Leu Leu Leu Trp Val Pro Gly Ser Thr Gly
            20                  25                  30

Asp Thr Val Leu Thr Gln Ser Pro Ala Leu Ala Val Ser Leu Gly Gln
        35                  40                  45

Arg Val Thr Ile Ser Cys Lys Ala Ser Glu Ser Val Ser Ser Ser Met
    50                  55                  60

Tyr Ser Tyr Met His Trp Tyr Gln Gln Lys Pro Gly Gln Gln Pro Lys
65                  70                  75                  80

Leu Leu Ile Tyr Arg Ala Ser Asn Leu Glu Ser Gly Val Pro Ala Arg
                85                  90                  95

Phe Ser Gly Ser Gly Ser Gly Thr Asp Phe Thr Leu Asn Ile Asp Pro
            100                 105                 110

Val Glu Ala Asp Asp Ile Ala Thr Tyr Phe Cys Gln Gln Ser Trp Asn
        115                 120                 125

Asp Pro Leu Thr Phe Gly Ser Gly Thr Lys Leu Glu Ile Lys Arg Ala
    130                 135                 140

Asp Ala Ala Pro Thr Val Ser Ile Phe Pro Pro Ser Met Glu Gln Leu
145                 150                 155                 160

Thr Ser Gly Gly Ala Thr Val Val Cys Phe Val Asn Asn Phe Tyr Pro
                165                 170                 175

Arg Asp Ile Ser Val Lys Trp Lys Ile Asp Gly Thr Glu Arg
            180                 185                 190

<210>  18
<211>  187
<212>  PRT
<213>  Mus musculus

<400>  18

Asp Trp Cys Ala Ala Met Asp Arg Leu Thr Ser Ser Phe Leu Leu Leu
1               5                   10                  15

Met Val Pro Ala Tyr Val Leu Ser Gln Val Thr Leu Lys Glu Ser Gly
            20                  25                  30

Pro Gly Ile Leu Gln Pro Ser Gln Thr Leu Ser Leu Thr Cys Ser Phe
        35                  40                  45

Ser Gly Phe Ser Leu Ser Thr Tyr Gly Met Cys Val Gly Trp Ile Arg
    50                  55                  60

Gln Ser Ser Gly Lys Gly Leu Glu Trp Leu Ala Asn Ile Trp Trp Asn
65                  70                  75                  80

Asp Asp Lys Tyr Tyr Asn Pro Ser Leu Lys Asn Arg Leu Thr Ile Ser
                85                  90                  95

Lys Asp Thr Ser Asn Asn Gln Ala Phe Leu Lys Ile Thr Asn Met Asp
            100                 105                 110

Thr Ala Asp Thr Ala Thr Tyr Tyr Cys Ala Arg Gly Thr Asn Trp Glu
        115                 120                 125

Leu Gly Tyr Trp Gly Gln Gly Val Met Val Thr Val Ser Ser Glu Ser
    130                 135                 140

Gln Ser Ser Pro Thr Val Phe Pro Leu Val Ser Cys Glu Ser Pro Leu
145                 150                 155                 160

Ser Asp Glu Asn Leu Val Ala Met Gly Cys Leu Ala Arg Asp Phe Leu
                165                 170                 175

Pro Ser Ser Ile Ser Phe Thr Trp Asn Tyr Gln

180                 185

<210>  19
<211>  190
<212>  PRT
<213>  Mus musculus

<400>  19

Met Gly Ile Leu Arg Ser Leu Arg Gly Ser Lys Met Leu Ala Ala His
1               5                   10                  15

Leu His Leu Leu Leu Leu Leu Cys Phe Thr Val Ser Asn Gly Gln Ile
            20                  25                  30

Met Leu Thr Gln Gln Ala Glu Ser Leu Trp Ile Ser Pro Gly Glu Arg
        35                  40                  45

Val Ser Ile Thr Cys Arg Ala Ser Gln Ser Leu Leu Tyr Thr Asp Gly
    50                  55                  60

Lys His Tyr Leu Ser Trp Tyr Gln Gln Arg Pro Gly Gln Thr Thr Lys
65                  70                  75                  80

Ala Leu Ile Tyr His Ala Ser Val Arg Thr Asp Gly Val Pro Thr Arg
                85                  90                  95

Phe Ile Gly Ser Gly Ser Gly Thr Glu Phe Thr Leu Ser Ile Glu His
            100                 105                 110

Val Gln Pro Glu Asp Phe Ala Ile Tyr Tyr Cys Leu Gln Thr Leu Lys
        115                 120                 125

Ser Pro Trp Thr Phe Gly Gly Gly Thr Lys Leu Glu Leu Lys Arg Ala
    130                 135                 140

Asp Ala Ala Pro Thr Val Ser Ile Phe Pro Pro Ser Met Glu Gln Leu
145                 150                 155                 160

Thr Ser Gly Gly Ala Thr Val Val Cys Phe Val Asn Asn Phe Tyr Pro
                165                 170                 175

Arg Asp Ile Ser Val Lys Trp Lys Ile Asp Gly Thr Glu Arg
            180                 185                 190

<210>  20
<211>  502

<212>  PRT
<213>  Rattus norvegicus

<400>  20

Leu His Ser Met Ala Val Leu Val Leu Leu Leu Cys Leu Val Thr Phe
1               5                   10                  15

Pro Ser Cys Val Leu Ser Gln Val Gln Leu Met Glu Ser Gly Pro Gly
            20                  25                  30

Leu Val Gln Pro Ser Glu Thr Leu Ser Leu Ile Cys Thr Val Ser Gly
        35                  40                  45

Phe Ser Leu Thr Ser Tyr Asn Val His Trp Val Arg Gln Pro Pro Gly
    50                  55                  60

Lys Gly Leu Glu Trp Met Gly Val Met Trp Ser Gly Gly Asn Thr Asp
65                  70                  75                  80

Tyr Asn Ser Ala Leu Lys Ser Arg Leu Ser Ile Ser Arg Asp Thr Ser
                85                  90                  95

Lys Asn Gln Val Phe Leu Lys Met Asn Ser Leu Gln Ser Glu Asp Ala
            100                 105                 110

Thr Thr Tyr Tyr Cys Ala Arg Glu Gly Tyr Pro Tyr Tyr Phe Asn Tyr
        115                 120                 125

Trp Gly Gln Gly Val Met Val Thr Val Ser Ser Ala Glu Thr Thr Ala
    130                 135                 140

Pro Ser Val Tyr Pro Leu Ala Pro Gly Thr Ala Leu Lys Ser Asn Ser
145                 150                 155                 160

Met Val Thr Leu Gly Cys Leu Val Lys Gly Tyr Phe Pro Glu Pro Val
                165                 170                 175

Thr Val Thr Trp Asn Ser Gly Ala Leu Ser Ser Gly Val His Thr Phe
            180                 185                 190

Pro Ala Val Leu Gln Ser Gly Leu Tyr Thr Leu Thr Ser Ser Val Thr
        195                 200                 205

Val Pro Ser Ser Thr Trp Ser Ser Gln Ala Val Thr Cys Asn Val Ala
    210                 215                 220

His Pro Ala Ser Ser Thr Lys Val Asp Lys Lys Ile Val Pro Arg Glu

225                 230                 235                 240

Cys Asn Pro Cys Gly Cys Thr Gly Ser Glu Val Ser Ser Val Phe Ile
                245                 250                 255

Phe Pro Pro Lys Thr Lys Asp Val Leu Thr Ile Thr Leu Thr Pro Lys
            260                 265                 270

Val Thr Cys Val Val Val Asp Ile Ser Gln Asn Asp Pro Glu Val Arg
        275                 280                 285

Phe Ser Trp Phe Ile Asp Asp Val Glu Val His Thr Ala Gln Thr His
    290                 295                 300

Ala Pro Glu Lys Gln Ser Asn Ser Thr Leu Arg Ser Val Ser Glu Leu
305                 310                 315                 320

Pro Ile Val His Arg Asp Trp Leu Asn Gly Lys Thr Phe Lys Cys Lys
                325                 330                 335

Val Asn Ser Gly Ala Phe Pro Ala Pro Ile Glu Lys Ser Ile Ser Lys
            340                 345                 350

Pro Glu Gly Thr Pro Arg Gly Pro Gln Val Tyr Thr Met Ala Pro Pro
        355                 360                 365

Lys Glu Glu Met Thr Gln Ser Gln Val Ser Ile Thr Cys Met Val Lys
    370                 375                 380

Gly Phe Tyr Pro Pro Asp Ile Tyr Thr Glu Trp Lys Met Asn Gly Gln
385                 390                 395                 400

Pro Gln Glu Asn Tyr Lys Asn Thr Pro Pro Thr Met Asp Thr Asp Gly
                405                 410                 415

Ser Tyr Phe Leu Tyr Ser Lys Leu Asn Val Lys Lys Glu Thr Trp Gln
            420                 425                 430

Gln Gly Asn Thr Phe Thr Cys Ser Val Leu His Glu Gly Leu His Asn
        435                 440                 445

His His Thr Glu Lys Ser Leu Ser His Ser Pro Gly Lys Ser Gln Ser
    450                 455                 460

Pro Val Ala Pro Leu Gly Leu Lys Asp Ala Asn Thr Tyr Leu Tyr His

465                 470                 475                 480

Leu Ser Leu Cys Lys Ser Thr Gln Leu Cys Leu Gly Thr Leu Gln Lys
                485                 490                 495

Lys Lys Lys Lys Lys Lys
            500

<210>  21
<211>  105
<212>  PRT
<213>  Mus musculus

<400>  21

Glu Ser Gln Ser Phe Pro Asn Val Phe Pro Leu Val Ser Cys Glu Ser
1               5                   10                  15

Pro Leu Ser Asp Lys Asn Leu Val Ala Met Gly Cys Leu Ala Arg Asp
            20                  25                  30

Phe Leu Pro Ser Thr Ile Ser Phe Thr Trp Asn Tyr Gln Asn Asn Thr
        35                  40                  45

Glu Val Ile Gln Gly Ile Arg Thr Phe Pro Thr Leu Arg Thr Gly Gly
    50                  55                  60

Lys Tyr Leu Ala Thr Ser Gln Val Leu Leu Ser Pro Lys Ser Ile Leu
65                  70                  75                  80

Glu Gly Ser Asp Glu Tyr Leu Val Cys Lys Ile His Tyr Gly Gly Lys
                85                  90                  95

Asn Arg Asp Leu His Val Pro Ile Pro
            100                 105

<210>  22
<211>  308
<212>  PRT
<213>  Mus musculus

<400>  22

His Thr Asp Met Gly Val Pro Thr Gln Leu Leu Gly Leu Leu Leu Leu
1               5                   10                  15

Trp Ile Thr Asp Ala Ile Cys Asp Ile Gln Met Thr Gln Ser Pro Ala
            20                  25                  30

Ser Leu Ser Ala Ser Leu Gly Glu Thr Val Ser Ile Glu Cys Leu Ala

35                  40                  45

Ser Glu Gly Ile Ser Asn Asp Leu Ala Trp Tyr Gln Gln Lys Ser Gly
    50                  55                  60

Lys Ser Pro Gln Leu Leu Ile Tyr Ala Ala Ser Arg Leu Gln Asp Gly
65                  70                  75                  80

Val Pro Ser Arg Phe Ser Gly Ser Gly Ser Gly Thr Arg Tyr Ser Leu
                85                  90                  95

Lys Ile Ser Gly Met Gln Pro Glu Asp Glu Ala Asp Tyr Phe Cys Gln
            100                 105                 110

Gln Ser Tyr Lys Tyr Pro Tyr Thr Phe Gly Ala Gly Thr Lys Leu Glu
        115                 120                 125

Leu Lys Arg Ala Asp Ala Ala Pro Thr Val Ser Ile Phe Pro Pro Ser
    130                 135                 140

Thr Glu Gln Leu Ala Thr Gly Gly Ala Ser Val Val Cys Leu Met Asn
145                 150                 155                 160

Asn Phe Tyr Pro Arg Asp Ile Ser Val Lys Trp Lys Ile Asp Gly Thr
                165                 170                 175

Glu Arg Arg Asp Gly Val Leu Asp Ser Val Thr Asp Gln Asp Ser Lys
            180                 185                 190

Asp Ser Thr Tyr Ser Met Ser Ser Thr Leu Ser Leu Thr Lys Ala Asp
        195                 200                 205

Tyr Glu Ser His Asn Leu Tyr Thr Cys Glu Val Val His Lys Thr Ser
    210                 215                 220

Ser Ser Pro Val Val Lys Ser Phe Asn Arg Asn Glu Cys Thr Gln Arg
225                 230                 235                 240

Ser Gly Ala Thr Cys Ser Pro Ala Pro Ser Asn Leu Pro Ser Gly Leu
                245                 250                 255

Gly Asp Phe Pro Thr Ser Asp Leu Pro Leu Leu Arg Cys Ser Lys Pro
            260                 265                 270

Pro Pro His Leu Ile Leu Leu Pro Phe Leu Gly Phe Asp His Ala Asn

        275                 280                 285

Ile Trp Gly Ile Leu Asn Lys Val Asn Leu Cys Thr Lys Lys Lys Lys
    290                 295                 300

Lys Lys Lys Lys
305

<210>  23
<211>  417
<212>  DNA
<213>  Mus musculus

<400>  23
atgggatgga gctgtatcat gctcttcttg gcagcaacag ctacaggtgt ccactcccag      60

gtccaactgc agcagcctgg ggctgagctt gtgaagcctg gggcttcagt gaagctgtcc     120

tgcaaggctt ctggctacac cttcaccagc tactggatgc actgggtgaa gcagaggcct     180

ggacgaggcc ttgagtggat tggaaggatt gatcctaata gtggtggtac taagtacaat     240

gagaagttca agagcaaggc cacactgact gtagacaaac cctccagcac agcctacatg     300

cagctcagca gcctgacatc tgaggactct gcggtctatt attgtgcaag atacgattac     360

tacggtagta gctactttga ctactggggc caaggcacca ctctcacagt ctcctca        417

<210>  24
<211>  139
<212>  PRT
<213>  Mus musculus

<400>  24

Met Gly Trp Ser Cys Ile Met Leu Phe Leu Ala Ala Thr Ala Thr Gly
1               5                   10                  15

Val His Ser Gln Val Gln Leu Gln Gln Pro Gly Ala Glu Leu Val Lys
            20                  25                  30

Pro Gly Ala Ser Val Lys Leu Ser Cys Lys Ala Ser Gly Tyr Thr Phe
        35                  40                  45

Thr Ser Tyr Trp Met His Trp Val Lys Gln Arg Pro Gly Arg Gly Leu
    50                  55                  60

Glu Trp Ile Gly Arg Ile Asp Pro Asn Ser Gly Gly Thr Lys Tyr Asn
65                  70                  75                  80

Glu Lys Phe Lys Ser Lys Ala Thr Leu Thr Val Asp Lys Pro Ser Ser

85                  90                  95

Thr Ala Tyr Met Gln Leu Ser Ser Leu Thr Ser Glu Asp Ser Ala Val
            100                 105                 110

Tyr Tyr Cys Ala Arg Tyr Asp Tyr Tyr Gly Ser Ser Tyr Phe Asp Tyr
        115                 120                 125

Trp Gly Gln Gly Thr Thr Leu Thr Val Ser Ser
    130                 135